# EUROPEAN PATENT SPECIFICATION

(11) **EP 3 302 519 B1**
(45) Date of publication and mention of the grant of the patent: **01.12.2021**
(21) Application number: 15807907.9
(22) Date of filing: 09.12.2015
(51) Int. Cl.: A61K 38/17, A61P 37/00

(54) **ORNITHODOROS MOUBATA COMPLEMENT INHIBITOR FOR USE IN THE TREATMENT OF ACUTE GRAFT VERSUS HOST DISEASE**
ORNITHODOROS-MOUBATA-KOMPLEMENTINHIBITOR ZUR VERWENDUNG BEI DER BEHANDLUNG VON AKUTER TRANSPLANTAT-WIRT-REAKTION
INHIBITEUR DU COMPLÉMENT D'ORNITHODOROS MOUBATA DESTINÉ À ÊTRE UTILISÉ DANS LE TRAITEMENT DE MALADIE DU GREFFON CONTRE L'HÔTE AIGUË

(30) Priority: 08.06.2015 WO PCT/EP2015/062742
(43) Date of publication of application: 11.04.2018
(73) Proprietor: Volution Immuno Pharmaceuticals SA, 3461-1211 Geneve 3 (CH)
(72) Inventor: WESTON-DAVIES, Wynne H, Cheltenham GL54 4EF (GB)
(74) Representative: Carpmaels & Ransford LLP
(86) International application number: PCT/EP2015/079172
(87) International publication number: WO 2016/198133

(56) References cited:
- WO-A1-2007/028968
- WO-A1-2008/029167
- WO-A1-2014/047500
- WO-A1-2015/185760
- SJ Machin ET AL: "Coversin, a small protein complement inhibitor, is a potential novel anti-thrombotic agent", , 30 April 2014 (2014-04-30), XP054975969, Retrieved from the Internet: URL:http://www.bloodmed-video.com/memorial -symposium/ [retrieved on 2015-07-13]
- WYNNE H WESTON-DAVIES ET AL: "Clinical and Immunological Characterisation of Coversin, a Novel Small Protein Inhibitor of Complement C5 with Potential As a Therapeutic Agent in PNH and Other Complement Mediated Disorders", 56TH ASH ANNUAL MEETING AND EXPOSITION, 17 December 2014 (2014-12-17), pages 1-2, XP055201591,
- Dimitrios C Mastellos ET AL: "Complement in paroxysmal nocturnal hemoglobinuria: exploiting our current knowledge to improve the treatment landscape - Author Manuscript", Expert Review of Hematology, 2 April 2015 (2015-04-02), pages 1-26, XP055201601, Retrieved from the Internet: URL:http://www.ncbi.nlm.nih.gov/pmc/articl es/PMC4383744/pdf/nihms674108.pdf [retrieved on 2015-07-10]
- Anonymous: "7th International Conference on Complement Therapeutics - Aegean Conferences", , 6 June 2014 (2014-06-06), pages 1-11, XP055201648, Retrieved from the Internet: URL:http://www.aegeanconferences.org/src/A pp/pages/view/Program_7thComp?conference=8 5 [retrieved on 2015-07-10]
- JUN-ICHI NISHIMURA ET AL: "Genetic Variants in C5 and Poor Response to Eculizumab", NEW ENGLAND JOURNAL OF MEDICINE, vol. 370, no. 7, 13 February 2014 (2014-02-13), pages 632-639, XP055201672, ISSN: 0028-4793, DOI: 10.1056/NEJMoa1311084
- ANJANA E CHANDRASEKHAR: "Complement C5", UCSD MOLECULE PAGES, vol. 1, no. 2, 1 January 2012 (2012-01-01) , XP055201719, DOI: 10.6072/H0.MP.A004240.01
- DOROTHEA ORTH-HÖLLER ET AL: "Inhibition of terminal complement activation in severe Shiga toxin-associated HUS - perfect example for a fast track from bench to bedside", EMBO MOLECULAR MEDICINE, vol. 3, no. 11, 23 November 2011 (2011-11-23), pages 617-619, XP055201728, ISSN: 1757-4676, DOI: 10.1002/emmm.201100169
- HEPBURN N J ET AL: "In vivo characterization and therapeutic efficacy of a C5-specific inhibitor from the soft tick Ornithodoros moubata", JOURNAL OF BIOLOGICAL CHEMISTRY, AMERICAN SOCIETY FOR BIOCHEMISTRY AND MOLECULAR BIOLOGY, US, vol. 282, no. 11, 16 March 2007 (2007-03-16), pages 8292-8299, XP002564815, ISSN: 0021-9258, DOI: 10.1074/JBC.M609858200 [retrieved on 2007-01-10]
- JINDRICH SOLTYS ET AL: "Novel complement inhibitor limits severity of experimentally myasthenia gravis", ANNALS OF NEUROLOGY, vol. 65, no. 1, 4 January 2009 (2009-01-04), pages 67-75, XP055201799, ISSN: 0364-5134, DOI: 10.1002/ana.21536
- A. BARRATT-DUE ET AL: "Ornithodoros moubata Complement Inhibitor Is an Equally Effective C5 Inhibitor in Pigs and Humans", THE JOURNAL OF IMMUNOLOGY, vol. 187, no. 9, 1 November 2011 (2011-11-01), pages 4913-4919, XP055201794, ISSN: 0022-1767, DOI: 10.4049/jimmunol.1101000
- ANA-LAURA CARRERA-MARIN ET AL: "ACR Meeting", ARTHRITIS & RHEUMATISM, vol. 63, no. S10, 9 October 2011 (2011-10-09), page S5, XP055201815, ISSN: 0004-3591, DOI: 10.1002/art.33310
- ROVERSI ET AL: "The Structure of OMCI, a Novel Lipocalin Inhibitor of the Complement System", JOURNAL OF MOLECULAR BIOLOGY, ACADEMIC PRESS, UNITED KINGDOM, vol. 369, no. 3, 11 May 2007 (2007-05-11), pages 784-793, XP022077570, ISSN: 0022-2836, DOI: 10.1016/J.JMB.2007.03.064
- FREDSLUND F ET AL: "Structure of and influence of a tick complement inhibitor on human complement component 5", NATURE IMMUNOLOGY, NATURE PUBLISHING GROUP, GB, vol. 9, no. 7, 1 July 2008 (2008-07-01), pages 753-760, XP003025036, ISSN: 1529-2908, DOI: 10.1038/NI.1625 cited in the application
- JULIEN ZUBER ET AL: "Use of eculizumab for atypical haemolytic uraemic syndrome and C3 glomerulopathies", NATURE REVIEWS NEPHROLOGY, vol. 8, no. 11, 2 October 2012 (2012-10-02), pages 643-657, XP055144872, ISSN: 1759-5061, DOI: 10.1038/nrneph.2012.214 cited in the application
- Nelson J Chao: "Clinical manifestations, diagnosis, and grading of acute graft-versus-host disease", , 4 May 2015 (2015-05-04), pages 1-13, XP055252893, Retrieved from the Internet: URL:http://www.uptodate.com/contents/clini cal-manifestations-diagnosis-and-grading-o f-acute-graft-versus-host-disease?topicKey =HEME%2F3549&elapsedTimeMs=4&view=print&di splayedView=full [retrieved on 2016-02-24]
- JODELE SONATA ET AL: "Eculizumab Therapy in Children with Severe Hematopoietic Stem Cell Transplantation-Associated Thrombotic Microangiop", BIOLOGY OF BLOOD AND MARROW TRANSPLANTATION, KLUGE CARDEN JENNINGS PUBLISHING, CHARLOTTESVILLE, VA, US, vol. 20, no. 4, 25 December 2013 (2013-12-25), pages 518-525, XP028636297, ISSN: 1083-8791, DOI: 10.1016/J.BBMT.2013.12.565
- Wynne H Weston-Davies ET AL: "Phase I Clinical Trial of Coversin, a novel C5 and LTB4 inhibitor", 7th International Conference on Complement Therapeutics - Aegean Conferences, 9 June 2014 (2014-06-09), pages 1-17, XP055735871, Athens

SEQUENCE LISTING
<110> VOLUTION IMMUNO PHARMACEUTICALS SA
<120> METHOD OF TREATMENT
<130> P067660WO
<150> 2015-06-08
   <151> PCT/EP2015/062742
<160> 14
<170> SeqWin2010, version 1.0
<210> 1
   <211> 507
   <212> DNA
   <213> Ornithodoros moubata
<400> 1
   atgctggttt tggtgaccct gattttctcc ttttctgcga acatcgcata tgctgacagc  60
   gaaagcgact gcactggaag cgaacctgtt gacgccttcc aagctttcag tgagggcaaa  120
   gaggcatatg tcctggtgag gtccacggat cccaaagcga gggactgctt gaaaggagaa  180
   ccagccggag aaaagcagga caacacgttg ccggtgatga tgacgtttaa gaatggcaca  240
   gactgggctt caaccgattg gacgtttact ttggacggcg caaaggtaac ggcaaccctt  300
   ggtaacctaa cccaaaatag ggaagtggtc tacgactcgc aaagtcatca ctgccacgtt  360
   gacaaggtcg agaaggaagt tccagattat gagatgtgga tgctcgatgc gggagggctt  420
   gaagtggaag tcgagtgctg ccgtcaaaag cttgaagagt tggcgtctgg caggaaccaa  480
   atgtatcccc atctcaagga ctgctag                                      507
<210> 2
   <211> 168
   <212> PRT
   <213> Ornithodoros moubata
<400> 2
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Asn Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Asn Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165
<210> 3
   <211> 453
   <212> DNA
   <213> Ornithodoros moubata
<400> 3
   gacagcgaaa gcgactgcac tggaagcgaa cctgttgacg ccttccaagc tttcagtgag  60
   ggcaaagagg catatgtcct ggtgaggtcc acggatccca aagcgaggga ctgcttgaaa  120
   ggagaaccag ccggagaaaa gcaggacaac acgttgccgg tgatgatgac gtttaagaat  180
   ggcacagact gggcttcaac cgattggacg tttactttgg acggcgcaaa ggtaacggca  240
   acccttggta acctaaccca aaatagggaa gtggtctacg actcgcaaag tcatcactgc  300
   cacgttgaca aggtcgagaa ggaagttcca gattatgaga tgtggatgct cgatgcggga  360
   gggcttgaag tggaagtcga gtgctgccgt caaaagcttg aagagttggc gtctggcagg  420
   aaccaaatgt atccccatct caaggactgc tag                                453
<210> 4
   <211> 150
   <212> PRT
   <213> Ornithodoros moubata
<400> 4
   Asp Ser Glu Ser Asp Cys Thr Gly Ser Glu Pro Val Asp Ala Phe Gln
   1               5                   10                  15
   Ala Phe Ser Glu Gly Lys Glu Ala Tyr Val Leu Val Arg Ser Thr Asp
               20                  25                  30
   Pro Lys Ala Arg Asp Cys Leu Lys Gly Glu Pro Ala Gly Glu Lys Gln
           35                  40                  45
   Asp Asn Thr Leu Pro Val Met Met Thr Phe Lys Asn Gly Thr Asp Trp
       50                  55                  60
   Ala Ser Thr Asp Trp Thr Phe Thr Leu Asp Gly Ala Lys Val Thr Ala
   65                  70                  75                  80
   Thr Leu Gly Asn Leu Thr Gln Asn Arg Glu Val Val Tyr Asp Ser Gln
                   85                  90                  95
   Ser His His Cys His Val Asp Lys Val Glu Lys Glu Val Pro Asp Tyr
               100                 105                 110
   Glu Met Trp Met Leu Asp Ala Gly Gly Leu Glu Val Glu Val Glu Cys
           115                 120                 125
   Cys Arg Gln Lys Leu Glu Glu Leu Ala Ser Gly Arg Asn Gln Met Tyr
       130                 135                 140
   Pro His Leu Lys Asp Cys
   145                 150
<210> 5
   <211> 450
   <212> DNA
   <213> Ornithodoros moubata
<210> 6
   <211> 149
   <212> PRT
   <213> Ornithodoros moubata
<400> 6
   Ser Glu Ser Asp Cys Thr Gly Ser Glu Pro Val Asp Ala Phe Gln Ala
   1               5                   10                  15
   Phe Ser Glu Gly Lys Glu Ala Tyr Val Leu Val Arg Ser Thr Asp Pro
               20                  25                  30
   Lys Ala Arg Asp Cys Leu Lys Gly Glu Pro Ala Gly Glu Lys Gln Asp
           35                  40                  45
   Asn Thr Leu Pro Val Met Met Thr Phe Lys Asn Gly Thr Asp Trp Ala
       50                  55                  60
   Ser Thr Asp Trp Thr Phe Thr Leu Asp Gly Ala Lys Val Thr Ala Thr
   65                  70                  75                  80
   Leu Gly Asn Leu Thr Gln Asn Arg Glu Val Val Tyr Asp Ser Gln Ser
                   85                  90                  95
   His His Cys His Val Asp Lys Val Glu Lys Glu Val Pro Asp Tyr Glu
               100                 105                 110
   Met Trp Met Leu Asp Ala Gly Gly Leu Glu Val Glu Val Glu Cys Cys
           115                 120                 125
   Arg Gln Lys Leu Glu Glu Leu Ala Ser Gly Arg Asn Gln Met Tyr Pro
       130                 135                 140
   His Leu Lys Asp Cys
   145
<210> 7
   <211> 447
   <212> DNA
   <213> Ornithodoros moubata
<400> 7
   gaaagcgact gcactggaag cgaacctgtt gacgccttcc aagctttcag tgagggcaaa  60
   gaggcatatg tcctggtgag gtccacggat cccaaagcga gggactgctt gaaaggagaa 120
   ccagccggag aaaagcagga caacacgttg ccggtgatga tgacgtttaa gaatggcaca 180
   gactgggctt caaccgattg gacgtttact ttggacggcg caaaggtaac ggcaaccctt 240
   ggtaacctaa cccaaaatag ggaagtggtc tacgactcgc aaagtcatca ctgccacgtt 300
   gacaaggtcg agaaggaagt tccagattat gagatgtgga tgctcgatgc gggagggctt 360
   gaagtggaag tcgagtgctg ccgtcaaaag cttgaagagt tggcgtctgg caggaaccaa 420
   atgtatcccc atctcaagga ctgctag                                      447
<210> 8
   <211> 148
   <212> PRT
   <213> Ornithodoros moubata
<400> 8
   Glu Ser Asp Cys Thr Gly Ser Glu Pro Val Asp Ala Phe Gln Ala Phe
   1               5                   10                  15
   Ser Glu Gly Lys Glu Ala Tyr Val Leu Val Arg Ser Thr Asp Pro Lys
               20                  25                  30
   Ala Arg Asp Cys Leu Lys Gly Glu Pro Ala Gly Glu Lys Gln Asp Asn
           35                  40                  45
   Thr Leu Pro Val Met Met Thr Phe Lys Asn Gly Thr Asp Trp Ala Ser
       50                  55                  60
   Thr Asp Trp Thr Phe Thr Leu Asp Gly Ala Lys Val Thr Ala Thr Leu
   65                  70                  75                  80
   Gly Asn Leu Thr Gln Asn Arg Glu Val Val Tyr Asp Ser Gln Ser His
                   85                  90                  95
   His Cys His Val Asp Lys Val Glu Lys Glu Val Pro Asp Tyr Glu Met
               100                 105                 110
   Trp Met Leu Asp Ala Gly Gly Leu Glu Val Glu Val Glu Cys Cys Arg
           115                 120                 125
   Gln Lys Leu Glu Glu Leu Ala Ser Gly Arg Asn Gln Met Tyr Pro His
       130                 135                 140
   Leu Lys Asp Cys
   145
<210> 9
   <211> 444
   <212> DNA
   <213> Ornithodoros moubata
<400> 9
   agcgactgca ctggaagcga acctgttgac gccttccaag ctttcagtga gggcaaagag  60
   gcatatgtcc tggtgaggtc cacggatccc aaagcgaggg actgcttgaa aggagaacca  120
   gccggagaaa agcaggacaa cacgttgccg gtgatgatga cgtttaagaa tggcacagac  180
   tgggcttcaa ccgattggac gtttactttg gacggcgcaa aggtaacggc aacccttggt  240
   aacctaaccc aaaataggga agtggtctac gactcgcaaa gtcatcactg ccacgttgac  300
   aaggtcgaga aggaagttcc agattatgag atgtggatgc tcgatgcggg agggcttgaa  360
   gtggaagtcg agtgctgccg tcaaaagctt gaagagttgg cgtctggcag gaaccaaatg  420
   tatccccatc tcaaggactg ctag                                         444
<210> 10
   <211> 147
   <212> PRT
   <213> Ornithodoros moubata
<400> 10
   Ser Asp Cys Thr Gly Ser Glu Pro Val Asp Ala Phe Gln Ala Phe Ser
   1               5                   10                  15
   Glu Gly Lys Glu Ala Tyr Val Leu Val Arg Ser Thr Asp Pro Lys Ala
               20                  25                  30
   Arg Asp Cys Leu Lys Gly Glu Pro Ala Gly Glu Lys Gln Asp Asn Thr
           35                  40                  45
   Leu Pro Val Met Met Thr Phe Lys Asn Gly Thr Asp Trp Ala Ser Thr
       50                  55                  60
   Asp Trp Thr Phe Thr Leu Asp Gly Ala Lys Val Thr Ala Thr Leu Gly
   65                  70                  75                  80
   Asn Leu Thr Gln Asn Arg Glu Val Val Tyr Asp Ser Gln Ser His His
                   85                  90                  95
   Cys His Val Asp Lys Val Glu Lys Glu Val Pro Asp Tyr Glu Met Trp
               100                 105                 110
   Met Leu Asp Ala Gly Gly Leu Glu Val Glu Val Glu Cys Cys Arg Gln
           115                 120                 125
   Lys Leu Glu Glu Leu Ala Ser Gly Arg Asn Gln Met Tyr Pro His Leu
       130                 135                 140
   Lys Asp Cys
   145
<210> 11
   <211> 441
   <212> DNA
   <213> Ornithodoros moubata
<400> 11
   gactgcactg gaagcgaacc tgttgacgcc ttccaagctt tcagtgaggg caaagaggca  60
   tatgtcctgg tgaggtccac ggatcccaaa gcgagggact gcttgaaagg agaaccagcc  120
   ggagaaaagc aggacaacac gttgccggtg atgatgacgt ttaagaatgg cacagactgg  180
   gcttcaaccg attggacgtt tactttggac ggcgcaaagg taacggcaac ccttggtaac  240
   ctaacccaaa atagggaagt ggtctacgac tcgcaaagtc atcactgcca cgttgacaag  300
   gtcgagaagg aagttccaga ttatgagatg tggatgctcg atgcgggagg gcttgaagtg  360
   gaagtcgagt gctgccgtca aaagcttgaa gagttggcgt ctggcaggaa ccaaatgtat  420
   ccccatctca aggactgcta g                                            441
<210> 12
   <211> 146
   <212> PRT
   <213> Ornithodoros moubata
<400> 12
   Asp Cys Thr Gly Ser Glu Pro Val Asp Ala Phe Gln Ala Phe Ser Glu
   1               5                   10                  15
   Gly Lys Glu Ala Tyr Val Leu Val Arg Ser Thr Asp Pro Lys Ala Arg
               20                  25                  30
   Asp Cys Leu Lys Gly Glu Pro Ala Gly Glu Lys Gln Asp Asn Thr Leu
           35                  40                  45
   Pro Val Met Met Thr Phe Lys Asn Gly Thr Asp Trp Ala Ser Thr Asp
       50                  55                  60
   Trp Thr Phe Thr Leu Asp Gly Ala Lys Val Thr Ala Thr Leu Gly Asn
   65                  70                  75                  80
   Leu Thr Gln Asn Arg Glu Val Val Tyr Asp Ser Gln Ser His His Cys
                   85                  90                  95
   His Val Asp Lys Val Glu Lys Glu Val Pro Asp Tyr Glu Met Trp Met
               100                 105                 110
   Leu Asp Ala Gly Gly Leu Glu Val Glu Val Glu Cys Cys Arg Gln Lys
           115                 120                 125
   Leu Glu Glu Leu Ala Ser Gly Arg Asn Gln Met Tyr Pro His Leu Lys
       130                 135                 140
   Asp Cys
   145
<210> 13
   <211> 438
   <212> DNA
   <213> Ornithodoros moubata
<400> 13
   tgcactggaa gcgaacctgt tgacgccttc caagctttca gtgagggcaa agaggcatat  60
   gtcctggtga ggtccacgga tcccaaagcg agggactgct tgaaaggaga accagccgga  120
   gaaaagcagg acaacacgtt gccggtgatg atgacgttta agaatggcac agactgggct  180
   tcaaccgatt ggacgtttac tttggacggc gcaaaggtaa cggcaaccct tggtaaccta  240
   acccaaaata gggaagtggt ctacgactcg caaagtcatc actgccacgt tgacaaggtc  300
   gagaaggaag ttccagatta tgagatgtgg atgctcgatg cgggagggct tgaagtggaa  360
   gtcgagtgct gccgtcaaaa gcttgaagag ttggcgtctg gcaggaacca aatgtatccc  420
   catctcaagg actgctag                                                438
<210> 14
   <211> 145
   <212> PRT
   <213> Ornithodoros moubata
<400> 14
   Cys Thr Gly Ser Glu Pro Val Asp Ala Phe Gln Ala Phe Ser Glu Gly
   1               5                   10                  15
   Lys Glu Ala Tyr Val Leu Val Arg Ser Thr Asp Pro Lys Ala Arg Asp
               20                  25                  30
   Cys Leu Lys Gly Glu Pro Ala Gly Glu Lys Gln Asp Asn Thr Leu Pro
           35                  40                  45
   Val Met Met Thr Phe Lys Asn Gly Thr Asp Trp Ala Ser Thr Asp Trp
       50                  55                  60
   Thr Phe Thr Leu Asp Gly Ala Lys Val Thr Ala Thr Leu Gly Asn Leu
   65                  70                  75                  80
   Thr Gln Asn Arg Glu Val Val Tyr Asp Ser Gln Ser His His Cys His
                   85                  90                  95
   Val Asp Lys Val Glu Lys Glu Val Pro Asp Tyr Glu Met Trp Met Leu
               100                 105                 110
   Asp Ala Gly Gly Leu Glu Val Glu Val Glu Cys Cys Arg Gln Lys Leu
           115                 120                 125
   Glu Glu Leu Ala Ser Gly Arg Asn Gln Met Tyr Pro His Leu Lys Asp
       130                 135                 140
   Cys
   145

## Description

### FIELD OF THE INVENTION

The present invention relates to treating and preventing acute Graft versus host disease (GVHD).

### BACKGROUND TO THE INVENTION

Graft versus host disease (GVHD) is believed to arise from a complex interaction between donor and recipient adaptive immunity. GVHD often occurs in humans after allogeneic stem-cell transplantation, but may also arise after transplantation of solid organs containing lymphoid tissue and transfusion of unirradiated blood products. Acute GVHD describes a distinctive syndrome of dermatitis, hepatitis, and enteritis developing within 100 days of treatment e.g. allogeneic hematopoietic-cell transplantation (HCT). Chronic GVHD describes a more diverse syndrome developing after day 100.

For GVHD to occur the following criteria must be met, discussed in [1]:
1. The graft must contain immunologically competent cells.
2. The host must possess transplantation alloantigens that are lacking in the donor graft so that the host appears foreign to the graft and can therefore stimulate it antigenically.
3. The host itself must be incapable of mounting an effective immunologic reaction against the graft, or it must at least allow for sufficient time for the graft to manifest its immunologic capabilities

Patient groups at risk for GVHD include allogenic HCT patients, such as those that are receiving no GVHD prophylaxis, older patients, recipients of HLA-non-identical stem cells and recipients of grafts. Solid-organ transplantation patients at risk for GVHD include recipients of small-bowel transplants (organs containing lymphoid tissue). Patients who are subject to transfusion of unirradiated blood products at particular risk include those that have congenital immunodeficiency syndromes, neonates and foetuses, those that are receiving immunosuppressive chemoradiotherapy and those that are receiving directed blood donations from partially HLA-identical, HLA-homologous donors.

Autologous GVHD may also occur. This occurs after autologous or syngeneic HCT and is believed to arise from tissue damage caused by treatment or by secondary cytokine production, which exposes cryptic self-antigens, which the immune system may newly recognize only after HCT. Transfusion-associated GVHD occurs 4-30 days after transfusion and resembles hyperacute GVHD after allogeneic HCT.

Acute GVHD may initially appear within 100 days of transplant. Hyperacute GVHD is GVHD that arises 7-14 days after transplantation. Acute GVHD may have involvement of the skin (a pruritic or painful rash), liver (e.g. asymptomatic elevation of bilirubin, alanine aminotransferase (ALT), aspartate aminotransferase (AST), and alkaline phosphatase levels similar to those observed with cholestatic jaundice, and ensuing pruritus with hyperbilirubinemia), or the intestine (e.g. the distal small bowel and colon, e.g. resulting in diarrhea, intestinal bleeding, cramping abdominal pain, and ileus, or the upper-GI, manifesting as anorexia and dyspepsia without diarrhea). Acute GVHD also has been associated with increased risk of infectious and noninfectious pneumonia and sterile effusions, hemorrhagic cystitis with infective agents, thrombocytopenia, and anemia. Hemolytic-uremic syndrome (thrombotic microangiopathy) has been observed in patients given cyclosporine A (CSP A) who developed severe GVHD.

Chronic GVHD is viewed as an extension of acute GVHD. However, it also may occur for the first time in patients who never have clinical evidence of acute GVHD, or it may emerge after a quiescent interval after acute GVHD resolves.

Various systems exist for staging and grading GVHD (see [2]):

**Table 1. Clinical Staging of Acute GVHD**

| **Stage** | **Skin Findings** | **Liver Findings (Bilirubin level, mg/dL)** | **Gut Findings** |
|---|---|---|---|
| + | Maculopapular rash on < 25% of body surface | 2-3 | Diarrhea 500-1000 mL/d or persistent nausea |
| ++ | Maculopapular rash on 25-50% of body surface | 3-6 | Diarrhea 1000-1500 mL/d |
| +++ | Generalized erythroderma | 6-15 | Diarrhea > 1500 mL/d |
| ++++ | Desquamation and bullae | >15 | Pain with or without ileus |

**Table 2. Clinical Grading of Acute GVHD**

| **Overall Grade** | **Stage** | | | |
|---|---|---|---|---|
| | **Skin** | **Liver** | **Gut** | **Functional Impairment** |
| 0 (None) | 0 | 0 | 0 | 0 |
| I (Mild) | + to ++ | 0 | 0 | 0 |
| II (Moderate) | + to +++ | + | + | + |
| III (Severe) | ++ to +++ | ++ to +++ | ++ to +++ | ++ |
| IV (Life-threatening) | ++ to ++++ | ++ to ++++ | ++ to ++++ | +++ |

Existing treatments for acute GVHD include prophylaxis and treatment as set out in Table 3 below:

**Table 3**

| Primary prophylaxis for acute GVHD | |
|---|---|
| Cyclosporine | Immune suppressant |
| Methotrexate | Immune suppressant |
| Tacrolimus | Immune suppressant |
| Treatment for acute GVHD | |
| Intravenously administered glucocorticoids e.g. prednisone | Immune suppressant |
| | |

PCT application PCT/EP2015/062742 (WO 2015/185760 A1) describes and claims methods of treating complement mediated disorders in patients who are resistant to eculizumab, and [3] also presents preliminary experimental work showing that when serum from an eculizumab resistant patient was spiked with Coversin and eculizumab, the dose response curve was normal for Coversin but not for eculizumab.

Sonata et al. (2014) "Eculizumab Therapy in Children with Severe Hematopoietic Stem Cell Transplantation-Associated Thrombotic Microangiopathy", Biol Blood Marrow Transplant, 518-525, describes treatment of acute GVHD which is concurrent with hematopoietic stem cell transplantation-associated thrombotic microangiopathy (HSCT-TMA), the HSCT-TMA being treated with eculizumab.

There is a need for treatments that improve on, or are at least alternatives to the known treatments for GVHD.

### SUMMARY OF THE INVENTION

The present inventors have found that in a patient suffering from GVHD, who had previously not responded to treatment with eculizumab, a C5 monoclonal antibody, the systemic administration of the tick protein Coversin (also referred to as EV576 and OmCI in the art and herein [19]) led to stabilisation of vital signs and reduced rectal bleeding. Systemically administered coversin can therefore be used to treat and prevent acute GVHD.

The invention provides an agent for use in treating or preventing acute GVHD in a subject, wherein the subject has not been treated with eculizumab, wherein the agent is administered systemically and wherein the agent is:
(i) a protein comprising amino acids 19 to 168 of the amino acid sequence in SEQ ID NO: 2 or a functional equivalent of this protein; or
(ii) a nucleic acid molecule encoding a protein as recited in (i),
wherein the functional equivalent is:
(a) a homologue of a protein as defined in (i) comprising or consisting of a sequence with greater than 80% identity to the sequence of amino acids 19 to 168 or amino acids 1 to 168 of the amino acid sequence SEQ ID NO: 2; or
(b) a fragment of a protein, wherein the protein has the amino acid sequence of SEQ ID NO: 2, or a fragment of a homologue, wherein the homologue has greater than 80% identity to the sequence of amino acids 19 to 168 or amino acids 1 to 168 of the amino acid sequence SEQ ID NO: 2,
wherein said functional equivalent retains the ability to bind complement C5 and to prevent the cleavage of complement C5 by C5 convertase into complement C5a and complement C5b-9.

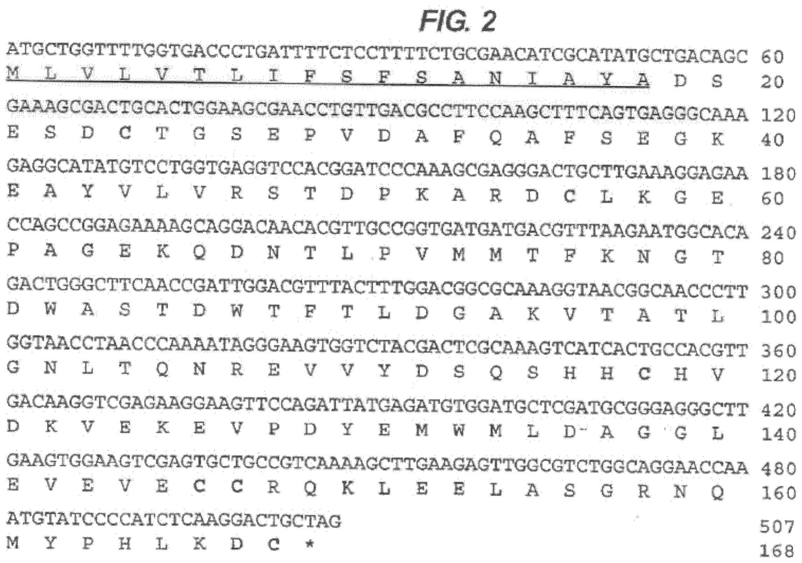

The disclosure (not according to the invention) provides a method of treating or preventing acute GVHD comprising systemically administering to a subject in need thereof a therapeutically or prophylactically effective amount of an agent which is a protein comprising amino acids 19 to 168 of the amino acid sequence in Figure 2 (SEQ ID NO: 2) or a functional equivalent of this protein.

The disclosure (not according to the invention) also provides an agent which is a protein comprising amino acids 19 to 168 of the amino acid sequence in Figure 2 (SEQ ID NO: 2) or a functional equivalent of this protein for treating or preventing acute GVHD in a subject, wherein the agent is administered systemically.

The disclosure (not according to the invention) also provides a method of treating or preventing acute GVHD comprising systemically administering to a subject in need thereof a therapeutically or prophylactically effective amount of an agent which is a nucleic acid molecule encoding a protein comprising amino acids 19 to 168 of the amino acid sequence in Figure 2 (SEQ ID NO: 2) or a functional equivalent of this protein.

The disclosure (not according to the invention) also provides an agent which is a nucleic acid molecule encoding a protein comprising amino acids 19 to 168 of the amino acid sequence in Figure 2 (SEQ ID NO: 2) or a functional equivalent of this protein for treating or preventing GVHD in a subject, wherein the agent is administered systemically.

### Complement

The complement system is an essential part of the body's natural defence mechanism against foreign invasion and is also involved in the inflammatory process. More than 30 proteins in serum and at the cell surface are involved in complement system function and regulation. Recently it has become apparent that, as well as the -35 known components of the complement system which may be associated with both beneficial and pathological processes, the complement system itself interacts with at least 85 biological pathways with functions as diverse as angiogenesis, platelet activation, glucose metabolism and spermatogenesis

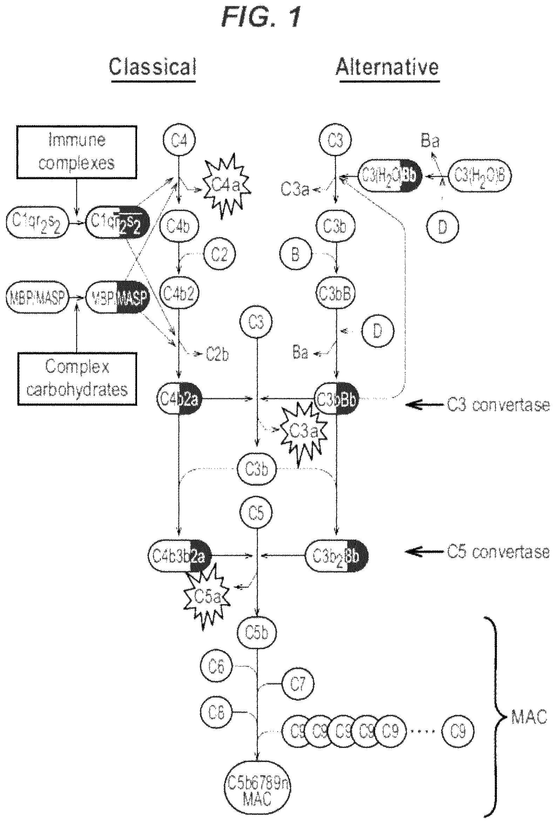

The complement system is activated by the presence of foreign antigens. Three activation pathways exist: (1) the classical pathway which is activated by IgM and IgG complexes or by recognition of carbohydrates; (2) the alternative pathway which is activated by non-self surfaces (lacking specific regulatory molecules) and by bacterial endotoxins; and (3) the lectin pathway which is activated by binding of mannan-binding lectin (MBL) to mannose residues on the surface of a pathogen. The three pathways comprise parallel cascades of events that result in the production of complement activation through the formation of similar C3 and C5 convertases on cell surfaces resulting in the release of acute mediators of inflammation (C3a and C5a) and formation of the membrane attack complex (MAC). The parallel cascades involved in the classical and alternative pathways are shown in Figure 1.

The classical complement pathway, the alternative complement pathway and the lectin complement pathway are herein collectively referred to as the complement pathways.

### Complement C5 polymorphisms

Several polymorphisms of human C5 have been reported [4- 8]. Mutations in the gene encoding C5 have been associated with various pathologies including complement component 5 deficiency, a disease where patients show a propensity for severe recurrent infections. Defects in this gene have also been linked to susceptibility to liver fibrosis and to rheumatoid arthritis. Polymorphisms in human C5 include insertions, deletions, single amino acid substitutions, frame-shifts, truncations and combinations of these changes.

Certain polymorphisms of human C5 alter C5 activity with clinical significance, e.g. polymorphisms affecting Arg885 of wildtype C5 include Arg885Cys (encoded by c.2653C>T) and p.Arg885His (encoded by c.2654G>A), both of which decrease the theraputic effectiveness of the mAb eculizumab [7].

The term "C5 polymorphism" is used herein to mean any variant of C5 other than the wild-type C5. In a human subject, the wild-type C5 is the C5 protein with accession number NP_001726.2 ; version GI:38016947. The term "C5 polymorphism" includes insertions, deletions, single or multiple amino acid substitutions, frame-shifts, truncations and combinations of these changes in the C5 protein.

These polymorphisms can be present as either heterozygous or homozygous polymorphisms, such as heterozygous C5 for a given polymorphism, homozygous for one polymorphism or heterozygous for different polymorphisms.

Polymorphisms include changes to the amino acid sequence of wildtype C5 which are in proximity to, or within the epitope for eculizumab, (i.e. 879KSSKC883, including K879, S880, S881, K882 and/or C883). For example, any change may be in the epitope for eculizumab or up to 10, 9, 8, 7, 6, 5, 4, 3, 2, 1 amino acids to the N- or C- terminus of the epitope for eculizumab.

Preferably, the amino acid change is not within or in proximity to the Coversin binding site of C5. This is believed to be a conserved region atop C5α at the distal end of the highly conserved CUB-C5d-MG8 superdomain of C5.

C5 polymorphisms may decrease the effectiveness of one or more agents that inhibit the classical complement pathway, the alternative complement pathway and the lectin complement pathway in a subject with wild-type C5. By "decrease the effectiveness" it is meant that the agent has an IC₅₀ for the polymorphic C5 protein that is at least 2, 3, 4, 5, 6, 7, 8, 9, 10, 20, 30, 40, 50, 100, 1000 or more times greater than the IC₅₀ of the same agent for the wild-type C5 protein.

The C5 polymorphism may decrease the effectiveness of one or more agents that inhibit the classical complement pathway, the alternative complement pathway and the lectin complement pathway, but does not decrease the effectiveness of Coversin or functional equivalents thereof. The C5 polymorphism may decrease the effectiveness of one or more anti-C5 monoclonal antibodies that inhibit the classical complement pathway, the alternative complement pathway and the lectin complement pathway in a subject with wild-type C5, but does not decrease the effectiveness of other agents that inhibit the classical complement pathway, the alternative complement pathway and the lectin complement pathway by binding to C5 without blocking the C5 convertase binding site.

By "does not decrease the effectiveness" it is meant that the IC₅₀ of Coversin or other agents that inhibit the classical complement pathway, the alternative complement pathway and the lectin complement pathway by binding to C5 without blocking the C5 convertase binding site, for the wild-type C5 protein is at least 75%, 80%, 85%, 90%, 95%, 96%, 97%, 98%, 99% or 100% of the IC₅₀ of Coversin or other agents that inhibit the classical complement pathway, the alternative complement pathway and the lectin complement pathway by binding to C5 without blocking the C5 convertase binding site for the polymorphic C5 protein. The term "does not decrease" also encompasses an increase in effectiveness.

In an alternative embodiment, effectiveness can be measured by measuring the ability of the agent to inhibit complement activation in serum taken from the subject. For example, complement activity in the serum of said subjects can be measured by any means known in the art or described herein, for example the haemolytic assays described in reference [9]. An agent would be considered to inhibit complement activity in said subject if complement activity in the presence of the agent is reduced when compared to a control. By "reduced" in this context it is meant that complement activity in the treated sample is at least 10, 20, 30, 40, 50, 60, 70, 80, 90, or 100%, reduced compared to a control.

The C5 polymorphism may decrease the effectiveness of monoclonal antibody agents in inhibiting activation of one or more of the complement pathways. The C5 polymorphism may decrease the effectiveness of the monoclonal antibody eculizumab in inhibiting activation of one or more of the complement pathways. The C5 polymorphism may decrease the effectiveness of agents that inhibit the classical complement pathway, the alternative complement pathway and the lectin complement pathway by blocking the C5 convertase binding site. The C5 polymorphism may be at position Arg885. Specific polymorphisms at this position include Arg885Cys or Arg885His.

Polymorphisms that alter binding the affinity of C5 to known anti-C5 monoclonal antibodies such as eculizumab, Pexelizumab, and/or N19-8, or the effectiveness of peptidic complement inhibitors such as ARC 1905 are also of interest.

The disclosure (not according to the invention) provides a method of treating or preventing acute GVHD comprising systemically administering to a subject in need thereof a therapeutically or prophylactically effective amount of an agent which is a protein comprising amino acids 19 to 168 of the amino acid sequence in Figure 2 (SEQ ID NO: 2) or a functional equivalent of this protein, and wherein the subject
i) does not have or has not been tested for a complement C5 polymorphism, e.g. a complement C5 polymorphism which decreases the effectiveness of monoclonal antibody agents in inhibiting activation of one or more of the complement pathways, such as a complement C5 polymorphism which decreases the effectiveness of the monoclonal antibody eculizumab in inhibiting activation of one or more of the complement pathways, and/or
ii) does not have or has not been tested for a complement C5 polymorphism which decreases the effectiveness of one or more agents that inhibit the classical complement pathway, the alternative complement pathway and the lectin complement pathway, but does not decrease the effectiveness of Coversin or functional equivalents thereof, and/or
iii) does not have or has not been tested for a complement C5 polymorphism which decreases the effectiveness of one or more agents that inhibit the classical complement pathway, the alternative complement pathway and the lectin complement pathway by blocking the C5 convertase site, but does not decrease the effectiveness of agents that inhibit the classical complement pathway, the alternative complement pathway and the lectin complement pathway without blocking the C5 convertase binding site, and/or
iv) does not have or has not been tested for resistance to monoclonal antibody agent treatment wherein the treatment is intended to inhibit activation of one or more of the complement pathways, preferably wherein the monoclonal antibody is eculizumab, and/or
v) does not have or has not been tested for a complement C5 polymorphisms which increases the affinity of another agent, such as eculizumab, for the C5 protein, and/or
vi) does not have or has not been tested for resistance to treatment with an agent that inhibit the classical complement pathway, the alternative complement pathway and the lectin complement pathway by blocking the C5 convertase binding site, but does not decrease the effectiveness of agents that inhibit the classical complement pathway, the alternative complement pathway and the lectin complement pathway without blocking the C5 convertase binding site, and/or
vii) has not been treated with a monoclonal antibody agent treatment wherein the monoclonal antibody is intended to inhibit activation of one or more of the complement pathways, preferably wherein the monoclonal antibody an anti C5 antibody, more preferably eculizumab, and/or
viii) has not been selected for treatment with the agent of the invention on the basis of decreased effectiveness in the subject of a different agent (e.g. an anti C5 antibody).

Specific examples of such polymorphisms and anti C5 antibodies are discussed above.

The invention also provides an agent which is a protein comprising amino acids 19 to 168 of the amino acid sequence in Figure 2 (SEQ ID NO: 2) or a functional equivalent of this protein as defined in the claims for use in treating or preventing acute GVHD in a subject, wherein the subject has not been treated with eculizumab, wherein the agent is administered systemically and wherein the subject:
i) does not have or has not been tested for a complement C5 polymorphism, e.g. a complement C5 polymorphism which decreases the effectiveness of monoclonal antibody agents in inhibiting activation of one or more of the complement pathways, such as a complement C5 polymorphism which decreases the effectiveness of the monoclonal antibody eculizumab in inhibiting activation of one or more of the complement pathways,
ii) does not have or has not been tested for a complement C5 polymorphism which decreases the effectiveness of one or more agents that inhibit the classical complement pathway, the alternative complement pathway and the lectin complement pathway, but does not decrease the effectiveness of Coversin or functional equivalents thereof, and/or
iii) does not have or has not been tested for a complement C5 polymorphism which decreases the effectiveness of one or more agents that inhibit the classical complement pathway, the alternative complement pathway and the lectin complement pathway by blocking the C5 convertase site, but does not decrease the effectiveness of agents that inhibit the classical complement pathway, the alternative complement pathway and the lectin complement pathway without blocking the C5 convertase binding site, and/or
iv) does not have or has not been tested for resistance to monoclonal antibody agent treatment wherein the treatment is intended to inhibit activation of one or more of the complement pathways, preferably wherein the monoclonal antibody is eculizumab, and/or
v) does not have or has not been tested for a complement C5 polymorphisms which increases the affinity of another agent, such as eculizumab, for the C5 protein, and/or
vi) does not have or has not been tested for resistance to treatment with an agent that inhibit the classical complement pathway, the alternative complement pathway and the lectin complement pathway by blocking the C5 convertase binding site, but does not decrease the effectiveness of agents that inhibit the classical complement pathway, the alternative complement pathway and the lectin complement pathway without blocking the C5 convertase binding site, and/or
vii) has not been treated with a monoclonal antibody agent treatment wherein the monoclonal antibody is intended to inhibit activation of one or more of the complement pathways, preferably wherein the monoclonal antibody an anti C5 antibody, and/or
viii) has not been selected for treatment with the agent of the invention on the basis of decreased effectiveness in the subject of a different agent (e.g. an anti C5 antibody).

Specific examples of such polymorphisms and anti C5 antibodies are discussed above.

The disclosure (not according to the invention) also provides a method of treating or preventing acute GVHD comprising systemically administering to a subject in need thereof a therapeutically or prophylactically effective amount of an agent which is a nucleic acid molecule encoding a protein comprising amino acids 19 to 168 of the amino acid sequence in Figure 2 (SEQ ID NO: 2) or a functional equivalent of this protein, and wherein the subject
i) does not have or has not been tested for a complement C5 polymorphism, e.g. a complement C5 polymorphism which decreases the effectiveness of monoclonal antibody agents in inhibiting activation of one or more of the complement pathways, such as a complement C5 polymorphism which decreases the effectiveness of the monoclonal antibody eculizumab in inhibiting activation of one or more of the complement pathways,
ii) does not have or has not been tested for a complement C5 polymorphism which decreases the effectiveness of one or more agents that inhibit the classical complement pathway, the alternative complement pathway and the lectin complement pathway, but does not decrease the effectiveness of Coversin or functional equivalents thereof, and/or
iii) does not have or has not been tested for a complement C5 polymorphism which decreases the effectiveness of one or more agents that inhibit the classical complement pathway, the alternative complement pathway and the lectin complement pathway by blocking the C5 convertase site, but does not decrease the effectiveness of agents that inhibit the classical complement pathway, the alternative complement pathway and the lectin complement pathway without blocking the C5 convertase binding site, and/or
iv) does not have or has not been tested for resistance to monoclonal antibody agent treatment wherein the treatment is intended to inhibit activation of one or more of the complement pathways, preferably wherein the monoclonal antibody is eculizumab, and/or
v) does not have or has not been tested for a complement C5 polymorphisms which increases the affinity of another agent, such as eculizumab, for the C5 protein, and/or
vi) does not have or has not been tested for resistance to treatment with an agent that inhibit the classical complement pathway, the alternative complement pathway and the lectin complement pathway by blocking the C5 convertase binding site, but does not decrease the effectiveness of agents that inhibit the classical complement pathway, the alternative complement pathway and the lectin complement pathway without blocking the C5 convertase binding site, and/or
vii) has not been treated with a monoclonal antibody agent treatment wherein the monoclonal antibody is intended to inhibit activation of one or more of the complement pathways, preferably wherein the monoclonal antibody an anti C5 antibody, more preferably wherein the monoclonal antibody is eculizumab, and/or
viii) has not been selected for treatment with the agent of the invention on the basis of decreased effectiveness in the subject of a different agent (e.g. an anti C5 antibody).

Specific examples of such polymorphisms and anti C5 antibodies are discussed above.

The invention also provides an agent which is a nucleic acid molecule encoding a protein comprising amino acids 19 to 168 of the amino acid sequence in Figure 2 (SEQ ID NO: 2) or a functional equivalent of this protein as defined in the claims for treating or preventing GVHD in a subject, wherein the subject has not been treated with eculizumab, wherein the agent is administered systemically, and wherein the subject
i) does not have or has not been tested for a complement C5 polymorphism, e.g. a complement C5 polymorphism which decreases the effectiveness of monoclonal antibody agents in inhibiting activation of one or more of the complement pathways, such as a complement C5 polymorphism which decreases the effectiveness of the monoclonal antibody eculizumab in inhibiting activation of one or more of the complement pathways,
ii) does not have or has not been tested for a complement C5 polymorphism which decreases the effectiveness of one or more agents that inhibit the classical complement pathway, the alternative complement pathway and the lectin complement pathway, but does not decrease the effectiveness of Coversin or functional equivalents thereof, and/or
iii) does not have or has not been tested for a complement C5 polymorphism which decreases the effectiveness of one or more agents that inhibit the classical complement pathway, the alternative complement pathway and the lectin complement pathway by blocking the C5 convertase site, but does not decrease the effectiveness of agents that inhibit the classical complement pathway, the alternative complement pathway and the lectin complement pathway without blocking the C5 convertase binding site, and/or
iv) does not have or has not been tested for a complement C5 polymorphisms which increases the affinity of another agent, such as eculizumab, for the C5 protein, and/or
v) does not have or has not been tested for resistance to monoclonal antibody agent treatment wherein the treatment is intended to inhibit activation of one or more of the complement pathways, preferably wherein the monoclonal antibody is eculizumab, and/or
vi) does not have or has not been tested for resistance to treatment with an agent that inhibit the classical complement pathway, the alternative complement pathway and the lectin complement pathway by blocking the C5 convertase binding site, but does not decrease the effectiveness of agents that inhibit the classical complement pathway, the alternative complement pathway and the lectin complement pathway without blocking the C5 convertase binding site, and/or
vii) has not been treated with a monoclonal antibody agent treatment wherein the monoclonal antibody is intended to inhibit activation of one or more of the complement pathways, preferably wherein the monoclonal antibody an anti C5 antibody, and/or
viii) has not been selected for treatment with the agent of the invention on the basis of decreased effectiveness in the subject of a different agent (e.g. an anti C5 antibody).

Specific examples of such polymorphisms and anti C5 antibodies are discussed above.

### C5 polymorphisms

As discussed above, subjects with two C5 polymorphisms at position Arg885 (c.2653C>T (p.Arg885Cys) and c.2654G>A (p.Arg885His)) do not respond to eculizumab, and such subjects are examples of subjects who are eculizumab resistant. Coversin has been shown to be able to inhibit C5 cleavage and activation of the complement pathways irrespective of the presence or absence of the polymorphisms because it interacts with complement C5 protein in a different manner to eculizumab. Coversin and eculizumab bind to C5 at different locations on the C5 molecule. Coversin binds to C5, which results in stabilization of the global conformation of C5 but does not block the C5a cleavage site [10].

The polymorphism Arg885His is particularly prevalent in subjects of Japanese and Han Chinese origin. Coversin can be used in a sub-population with these ethnic origins. Coversin may also be used in populations of other ethnic origins, e.g. those who are not of Japanese origin or Han Chinese origin and/or those who are not Caucasian. Suitable ethnic groups include the Afro-Caribbean, Slavic Caucasian, Non-Slavic Caucasian, Oriental Asiatic and Non Oriental Asiatic groups.

Subjects with C5 polymorphisms can be identified by routine techniques including molecular genetic analysis of the gene encoding the C5 protein including sequencing of the gene [7]; testing the ability of various agent to inhibit complement activation in the subject as described herein or by other methods known in the art; and/or biochemical analysis of the C5 protein from the subject, including isoelectric focusing and functional detection [11]. In a clinical setting, a subject with a C5 polymorphism may be identified by an unexpectedly poor response to an agent that inhibits the classical complement pathway, the alternative complement pathway and the lectin complement pathway.

Complement activity in the serum of said subjects can be measured by any means known in the art or described herein, for example the haemolytic assays described in reference [12] and/or by using the Quidel CH50 method as referred to in the example. An agent would be considered to inhibit complement activity in said subject if complement activity in the presence of the agent is reduced when compared to a control. By "reduced" in this context it is meant that complement activity in the treated sample is at least 10, 20, 30, 40, 50, 60, 70, 80, 90, 100%, reduced compared to a control.

### Complement-mediated diseases and disorders

Activation of complement must be tightly controlled to prevent damage to the body's own tissues. Failure to control complement activation has been shown to play a role in a variety of diseases including, amongst others, acute pancreatitis, Age Related Macular Degeneration (AMD), atypical haemolytic uremeic syndrome (aHUS), Alzheimer's disease, Huntingdon's disease, Parkinson's disease, allergic encephalomyelitis, allotransplatation, asthma, adult respiratory distress syndrome, influenza, burn injuries, Crohn's disease, glomerulonephritis, haemolytic anaemia, haemodialysis, hereditary angioedema, ischaemia reperfusion injuries, multiple system organ failure, multiple sclerosis, myasthenia gravis, myocardial infarction, paroxysmal nocturnal haemoglobinuria (PNH), psoriasis, rheumatoid arthritis, septic shock, systemic lupus erythematosus, stroke, thrombotic thrombocytopaenicpurpura (TTP), traumatic brain injury, vascular leak syndrome, and transplantation rejection and graft versus host disease (GvHD), as well as various other peripheral nerve disorders and respiratory disorders [13- 18].

The invention concerns an agent for use in the treatment of acute GVHD in a subject, as defined in the claims.

The subject may be suffering from acute GVHD, may have GVHD with one or more symptoms that are at stage +, ++, +++ or ++++ and/or the subject may have a clinical grading of I, II, III or IV [2]. The outcome of the treatment of the invention may be an improvement in the stage and/or grade of the GVHD.

The subject suffering from acute GVHD may have tissue damage, e.g. internal (such as intestinal) tissue damage arising from the GVHD. As such, the outcome of the treatment of the invention may be a reduction in this tissue damage.

Symptoms of GVHD can be measured by serum LDH. As such the outcome of the treatment of the invention may be a reduction in serum LDH e.g. as measured by standard methods known in the art.

The subject may have a reduced platelet count. As such the outcome of the treatment of the invention may be an increase in platelet count, e.g. as measured by standard methods known in the art.

Thus in a further embodiment of the disclosure (not according to the invention), there is provided a method of reducing tissue damage in a subject with acute GVHD, comprising systemically administering to a subject in need thereof a therapeutically or prophylactically effective amount of an agent which is a protein comprising amino acids 19 to 168 of the amino acid sequence in Figure 2 (SEQ ID NO: 2) or a functional equivalent of this protein, or which is a nucleic acid molecule encoding the protein or functional equivalent thereof. In the invention, there is also provided an agent which is a protein comprising amino acids 19 to 168 of the amino acid sequence in Figure 2 (SEQ ID NO: 2) or a functional equivalent of this protein as defined in the claims, or which is a nucleic acid molecule encoding the protein or functional equivalent thereof as defined in the claims, for reducing tissue damage in a subject with acute GVHD in a subject, wherein the subject has not been treated with eculizumab, and wherein the agent is administered systemically.

Thus in a further embodiment of the disclosure (not according to the invention), there is provided a method of reducing serum LDH in a subject with acute GVHD, comprising systemically administering to a subject in need thereof a therapeutically or prophylactically effective amount of an agent which is a protein comprising amino acids 19 to 168 of the amino acid sequence in Figure 2 (SEQ ID NO: 2) or a functional equivalent of this protein, or which is a nucleic acid molecule encoding the protein or functional equivalent thereof. In the invention, there is also provided an agent which is a protein comprising amino acids 19 to 168 of the amino acid sequence in Figure 2 (SEQ ID NO: 2) or a functional equivalent of this protein as defined in the claims, or which is a nucleic acid molecule encoding the protein or functional equivalent thereof as defined in the claims, for reducing serum LDH in a subject with acute GVHD in a subject, wherein the subject has not been treated with eculizumab, and wherein the agent is administered systemically.

Thus in a further embodiment of the disclosure (not according to the invention), there is provided a method of increasing platelet count in a subject with acute GVHD, comprising systemically administering to a subject in need thereof a therapeutically or prophylactically effective amount of an agent which is a protein comprising amino acids 19 to 168 of the amino acid sequence in Figure 2 (SEQ ID NO: 2) or a functional equivalent of this protein, or which is a nucleic acid molecule encoding the protein or functional equivalent thereof. In the invention, there is also provided an agent which is a protein comprising amino acids 19 to 168 of the amino acid sequence in Figure 2 (SEQ ID NO: 2) or a functional equivalent of this protein as defined in the claims, or which is a nucleic acid molecule encoding the protein or functional equivalent thereof as defined in the claims, for increasing platelet count in a subject with acute GVHD in a subject, wherein the subject has not been treated with eculizumab, and wherein the agent is administered systemically.

Preferred subjects, agents, doses and the like are as disclosed herein. Any reference to any reduction or increase is a reduction or increase compared to said subject in the absence of the treatment. Preferably, the parameter can be quantitated and where this is the case the increase or decrease is preferably statistically significant. For example the increase or decrease may be at least 3, 5, 10, 15, 20, 30, 40, 50% compared to the parameter in the absence of treatment.

The subject to which the agent of the invention is administered is preferably a mammal, preferably a human. The subject may be an adult, a child, or an infant. The subject to which the agent is administered is suffering from acute GVHD.

The subject may be e.g. at least 5 years old, or at least 6, 7, 8, 9, 10, 15, 20, 25, 30, 35, 40, 50, 60, 70 years old. The subject may be less than 4 years old, less than 3, 2, or 1 year old.

Subjects of interest are those with acute GVHD, as described herein. Preferred subjects may, for example have one or more of the following attributes:
the subject has acute GVHD following allogenic hamematopoetic cell transplant (HCT),
the subject has received no GVHD prophylaxis,
the subject has received HLA-nonidentical stem cells,
the subject has acute GVHD following solid-organ transplantation, e.g a small-bowel transplant, or following transfusion of unirradiated blood products,
the subject has acute GVHD after autologous or syngeneic HCT,
the subject has hyperacute GVHD.

### Agent to be used in the invention

According to one embodiment of the invention, the agent is a protein comprising amino acids 19 to 168 of the amino acid sequence in Figure 2 (SEQ ID NO: 2) or is a functional equivalent of this protein as defined in the claims. The agent may be a protein consisting of amino acids 19 to 168 of the amino acid sequence in Figure 2 or be a functional equivalent of this protein as defined in the claims.

According to an alternative embodiment, the protein used according to this embodiment of the invention may comprise or consist of amino acids 1 to 168 of the amino acid sequence in Figure 2 (SEQ ID NO: 2), or be a functional equivalent thereof as defined in the claims. The first 18 amino acids of the protein sequence given in Figure 2 form a signal sequence which is not required for C5 binding or for LTB4 binding activity and so this may optionally be dispensed with, for example, for efficiency of recombinant protein production.

The protein having the amino acid sequence given in Figure 2, also referred to herein as the Coversin protein, was isolated from the salivary glands of the tick *Ornithodoros moubata.* Coversin is an outlying member of the lipocalin family and is the first lipocalin family member shown to inhibit complement activation. The Coversin protein inhibits the alternative, classical and lectin complement pathways by binding C5 and preventing its cleavage by C5 convertase into Complement C5a and Complement C5b - 9, thus inhibiting both the action of C5a peptide and the MAC. The Coversin protein also binds LTB4. The term "Coversin protein", as used herein, refers to the sequence given in Figure 2 with or without the signal sequence.

The Coversin protein and the ability of this protein to inhibit complement activation has been disclosed in [19], where the Coversin protein was referred to as the "OmCI protein". The Coversin protein has also been shown to be effective in the treatment of myasthenia gravis [15], respiratory disorders [16] and peripheral nerve disorders [17]. The ability of the Coversin protein to bind eicosanoids including LTB4 and its use in the treatment of diseases mediated by a leukotriene or hydroxyeicosanoid has been suggested in [20].

It has now been found that the Coversin protein is effective in the treatment and prevention of acute GVHD in subjects. The data presented herein demonstrate that, in a subject with GVHD, complement activity *in vitro* was completely sensitive to Coversin, with 100% inhibition at all concentrations tested, and the subject furthermore showed an encouraging clinical response, with stabilisation of vital signs and reduced rectal bleeding.

According to a further embodiment of the invention, the agent may be a nucleic acid molecule encoding the Coversin protein or a functional equivalent thereof as defined in the claims. For example, gene therapy may be employed to effect the endogenous production of the Coversin protein by the relevant cells in the subject, either *in vivo* or *ex vivo.* Another approach is the administration of "naked DNA" in which the therapeutic gene is directly injected into the bloodstream or into muscle tissue.

Preferably, such a nucleic acid molecule comprises or consists of bases 55 to 507 of the nucleotide sequence in Figure 2 (SEQ ID NO: 1). This nucleotide sequence encodes the Coversin protein in Figure 2 without the signal sequence. The first 54 bases of the nucleotide sequence in Figure 2 encode the signal sequence which is not required for complement inhibitory activity or LTB4 binding activity. Alternatively, the nucleic acid molecule may comprise or consist of bases 1 to 507 of the nucleic acid sequence in Figure 2, which encodes the protein with the signal sequence.

The Coversin protein has been demonstrated to bind to C5 and prevent its cleavage by C5 convertase in rat, mouse and human serum with an IC₅₀ of approximately 0.02mg/ml. Functional equivalents of the Coversin protein preferably retain the ability to bind C5 with an IC₅₀ of less than 0.2 mg/ml, preferably less than 0.1 mg/ml, preferably less than 0.05 mg/ml, preferably less than 0.02 mg/ml, preferably less than 1µg/ml, preferably less than 100ng/ml, preferably less than 10ng/ml, more preferably still, less than 1ng/ml.

The Coversin protein has also been demonstrated to bind LTB4. Functional equivalents of the Coversin protein may also retain the ability to bind LTB4 with a similar affinity as the Coversin protein.

In one aspect of the invention, the agent binds complement C5, optionally complement C5 from subjects with complement C5 polymorphisms. The agent acts to prevent the cleavage of complement C5, optionally complement C5 from subjects with complement C5 polymorphisms, by C5 convertase into complement C5a and complement C5b-9. The agent may act to reduce C5a levels in a subject compared to an untreated subject. Functional equivalents of the agent also preferably share these properties.

In one aspect of the invention, the agent inhibits the classical complement pathway, the alternative complement pathway and the lectin complement pathway. In one aspect the agent binds to C5 in such a way as to stabilize the global conformation of C5 but not block the C5 convertase cleavage site. Binding of Coversin to C5 results in stabilization of the global conformation of C5 but does not block the convertase cleavage site. Functional equivalents of the agent also preferably share these properties.

The complement C5 protein, also referred to herein as C5, is cleaved by the C5 convertase enzyme (Figure 1). The products of this cleavage include an anaphylatoxin C5a and a lytic complex C5b - 9 also known as membrane attack complex (MAC). C5a is a highly reactive peptide implicated in many pathological inflammatory processes including neutrophil and eosinophil chemotaxis, neutrophil activation, increased capillary permeability and inhibition of neutrophil apoptosis [21].

MAC is associated with other important pathological processes including rheumatoid arthritis [22;23], proliferative glomerulonephritis [24], idiopathic membranous nephropathy [25], proteinurea [26], demyelination after acute axonal injury [27] and is also responsible for acute graft rejection following xenotransplantation [28].

Monoclonal antibodies and small molecules that bind and inhibit C5 have been developed to treat various diseases [14], in particular PNH, psoriasis, rheumatoid arthritis, systemic lupus erythematosus and transplant rejection. However, these monoclonal antibodies do not bind to certain C5 proteins from subjects with C5 polymorphisms, and are thus ineffective in these subjects [7]. In contrast, the Coversin agents, and functional equivalents thereof, inhibit complement C5 cleavage both in subjects with wild-type C5 and in subjects with C5 polymorphisms.

The ability of an agent to bind C5, including C5 from subjects with C5 polymorphisms, may be determined by standard *in vitro* assays known in the art, for example by western blotting following incubation of the protein on the gel with labelled C5. Preferably, the agent according to the invention binds C5, either wild-type and/or C5 from subjects with C5 polymorphisms, with an IC₅₀ of less than 0.2 mg/ml, preferably less than 0.1 mg/ml, preferably less than 0.05 mg/ml, preferably less than 0.04 mg/ml, preferably less than 0.03 mg/ml, preferably 0.02 mg/ml, preferably less than µg/ml, preferably less than 100ng/ml, preferably less than 10ng/ml, more preferably still, less than 1ng/ml. The agent need not have the same affinity for wild-type C5 and C5 from subjects with C5 polymorphisms. It may show higher, lower or the same affinity for wild-type C5 and C5 from subjects with C5 polymorphisms.

The ability of an agent to inhbit complement activation may be determined by measuring the ability of the agent to inhibit complement activation in serum. For example, complement activity in the serum can be measured by any means known in the art or described herein.

The agent may also be defined as having the function of inhibiting eicosanoid activity.

The agent according to this aspect of the invention may inhibit leukotrine B4 (LTB4) activity. In particular, the agent according to this aspect of the invention may bind LTB4. The ability of an agent to bind LTB4 may be determined by standard *in vitro* assays known in the art, for example by means of a competitive ELISA between coversin and anti-LTB4 antibody competing for binding to labelled LTB4. The agent according to the invention may bind LTB4 with an IC₅₀ of less than 0.2 mg/ml, preferably less than 0.1 mg/ml, preferably less than 0.05 mg/ml, preferably less than 0.04 mg/ml, preferably less than 0.03 mg/ml, preferably 0.02 mg/ml, preferably less than 1µg/ml, preferably less than 100ng/ml, preferably less than 10ng/ml, more preferably still, less than 1ng/ml. Functional equivalents of the agent also preferably share these properties.

According to one embodiment of the invention, the agent may bind all of C5, C5 from subjects with C5 polymorphisms, and LTB4. The agent according to this embodiment may thus act to prevent the cleavage of complement C5 by C5 convertase into complement C5a and complement C5b-9 (MAC), and also to inhibit LTB4 activity. Using an agent which binds to both C5 and LTB4 is particularly advantageous. C5 and the eicosanoid pathway are both believed to contribute to the observed pathology in many complement mediated diseases and disorders. Thus by using a single agent which inhibits multiple pathways involved in the inflammatory effects of complement-mediated diseases and disorders, an enhanced effect can be achieved, compared to using an agent which inhibits only a single pathway involved in the inflammatory effects of complement-mediated diseases and disorders. There are furthermore practical advantages associated with administering a single molecule.

Preferably, the agent of the invention is derived from a haematophagous arthropod. The term "haematophagous arthropod" includes all arthropods that take a blood meal from a suitable host, such as insects, ticks, lice, fleas and mites. Preferably, the agent is derived from a tick, preferably from the tick *Ornithodoros moubata.*

In one respect, the term "functional equivalent" is used herein to describe homologues and fragments of the Coversin protein which: a) retain its ability to bind C5, either wild-type C5 or C5 from a subject with a C5 polymorphism, and to prevent the cleavage of complement C5 by C5 convertase into complement C5a and complement C5b-9; and/or b) retain its ability to bind LTB4.

The term "functional equivalent" also refers to molecules that are structurally similar to the Coversin protein or that contain similar or identical tertiary structure, particularly in the environment of the active site or active sites of the Coversin protein that binds to C5, either wild-type C5 or C5 from a subject with a C5 polymorphism, and/or LTB4, such as synthetic molecules. Amino acids in Coversin that are likely to be required for LTB4 binding are described in [20].

The term "homologue" is meant to include reference to paralogues and orthologues of the Coversin sequence that is explicitly identified in Figure 2, including, for example, the Coversin protein sequence from other tick species, including *Rhipicephalus appendiculatus, R. sanguineus, R. bursa, A. americanum, A. cajennense, A. hebraeum, Boophilus microplus, B. annulatus, B. decoloratus, Dermacentor reticulatus, D. andersoni, D. marginatus, D. variabilis, Haemaphysalis inermis, Ha. leachii, Ha. punctata, Hyalomma anatolicum anatolicum, Hy. dromedarii, Hy. marginatum marginatum, Ixodes ricinus, I. persulcatus, I. scapularis, I. hexagonus, Argas persicus, A. reflexus, Ornithodoros erraticus, O. moubata moubata, O. m. porcinus, and O. savignyi.* The term "homologue" is also meant to include the equivalent Coversin protein sequence from mosquito species, including those of the *Culex, Anopheles* and *Aedes* genera, particularly *Culex quinquefasciatus, Aedes aegypti* and *Anopheles gambiae;* flea species, such as *Ctenocephalides felis* (the cat flea); horseflies; sandflies; blackflies; tsetse flies; lice; mites; leeches; and flatworms. The native Coversin protein is thought to exist in *O*. *moubata* in another three forms of around 18kDa and the term "homologue" is meant to include these alternative forms of Coversin.

Methods for the identification of homologues of the Coversin sequence given in Figure 2 will be clear to those of skill in the art. For example, homologues may be identified by homology searching of sequence databases, both public and private. Conveniently, publicly available databases may be used, although private or commercially-available databases will be equally useful, particularly if they contain data not represented in the public databases. Primary databases are the sites of primary nucleotide or amino acid sequence data deposit and may be publicly or commercially available. Examples of publicly-available primary databases include the GenBank database (http://www.ncbi.nlm.nih.gov/), the EMBL database (http://www.ebi.ac.uk/), the DDBJ database (http://www.ddbj.nig.ac.jp/), the SWISS-PROT protein database (http://expasy.hcuge.ch/), PIR (http://pir.georgetown.edu/), TrEMBL (http://www.ebi.ac.uk/), the TIGR databases (see http://www.tigr.org/tdb/index.html), the NRL-3D database (http://www.nbrfa.georgetown.edu), the Protein Data Base (http://www.rcsb.org/pdb), the NRDB database (ftp://ncbi.nlm.nih.gov/pub/nrdb/README), the OWL database (http://www.biochem.ucl.ac.uk/bsm/dbbrowser/OWL/) and the secondary databases PROSITE (http://expasy.hcuge.ch/sprot/prosite.html), PRINTS (http://iupab.leeds.ac.uk/bmb5dp/prints.html), Profiles (http://ulrec3.unil.ch/software/PFSCAN_form.html),

Pfam (http://www.sanger.ac.uk/software/pfam), Identify (http://dna.stanford.edu/identify/) and Blocks (http://www.blocks.fhcrc.org) databases. Examples of commercially-available databases or private databases include PathoGenome (Genome Therapeutics Inc.) and PathoSeq (previously of Incyte Pharmaceuticals Inc.).

Typically, greater than 30% identity between two polypeptides (preferably, over a specified region such as the active site) is considered to be an indication of functional equivalence and thus an indication that two proteins are homologous. Preferably, proteins that are homologues have a degree of sequence identity with the Coversin protein sequence identified in Figure 2 (SEQ ID NO:2) of greater than 60%. More preferred homologues have degrees of identity of greater than 80%, 90%, 95%, 98% or 99%, respectively with the Coversin protein sequence given in Figure 2 (SEQ ID NO:2). Percentage identity, as referred to herein, is as determined using BLAST version 2.1.3 using the default parameters specified by the NCBI (the National Center for Biotechnology Information; http://www.ncbi.nlm.nih.gov/) [Blosum 62 matrix; gap open penalty=11 and gap extension penalty= 1].

Functional equivalents of the Coversin protein sequence given in Figure 2 include mutants containing amino acid substitutions, insertions or deletions from the wild type sequence, for example, of 1, 2, 3, 4, 5, 7, 10 or more amino acids, or up to 1, 2, 3, 4, 5, 7 or 10 amino acids, provided that such mutants retain the ability to bind wild-type C5 and/or C5 from subjects with a C5 polymorphism. Mutants thus include proteins containing conservative amino acid substitutions that do not affect the function or activity of the protein in an adverse manner. This term is also intended to include natural biological variants (e.g. allelic variants or geographical variations within the species from which the Coversin proteins are derived). Mutants with improved ability to bind wild-type C5 and/or C5 from subjects with a C5 polymorphism and/or LTB4 may also be designed through the systematic or directed mutation of specific residues in the protein sequence.

Fragments of the Coversin protein and of homologues of the Coversin protein are also embraced by the term "functional equivalents" providing that such fragments retain the ability to bind wild-type C5 and/or C5 from subjects with a C5 polymorphism and/or LTB4. Fragments may include, for example, polypeptides derived from the Coversin protein sequence which are less than 150 amino acids, less than 125 amino acids, less than 100 amino acids, less than 75 amino acids, less than 50 amino acids, or even 25 amino acids or less, provided that these fragments retain the ability to bind to complement wild-type C5 and/or C5 from subjects with a C5 polymorphism and/or LTB4. Fragments may include, for example, polypeptides derived from the Coversin protein sequence which are at least 150 amino acids, at least 125 amino acids, at least 100 amino acids, at least 75 amino acids, at least 50 amino acids, or at least 25 amino acids, provided that these fragments retain the ability to bind to complement wild-type C5 and/or C5 from subjects with a C5 polymorphism and/or LTB4.

Any functional equivalent or fragment thereof preferably retains the pattern of cysteine residues that is found in Coversin. For example, said functional equivalent comprises six cysteine residues that are spaced relative to each other at a distance of 32 amino acids apart, 62 amino acids apart, 28 amino acids apart, 1 amino acid apart and 21 amino acids apart as arranged from the amino terminus to the carboxyl terminus of the sequence according to amino acids 1 to 168 of the amino acid sequence in Figure 2 (SEQ ID NO: 2). Exemplary fragments of Coversin protein are disclosed in SEQ ID NO: 4, SEQ ID NO: 6, SEQ ID NO: 8, SEQ ID NO: 10, SEQ ID NO: 12, SEQ ID NO: 14. The DNA encoding the corresponding fragments are disclosed in SEQ ID NO: 3, SEQ ID NO: 5, SEQ ID NO: 7, SEQ ID NO: 9, SEQ ID NO: 11, SEQ ID NO: 13.

Included as such fragments are not only fragments of the *O*. *moubata* Coversin protein that is explicitly identified herein in Figure 2, but also fragments of homologues of this protein, as described above. Such fragments of homologues will typically possess greater than 60% identity with fragments of the Coversin protein sequence in Figure 2, although more preferred fragments of homologues will display degrees of identity of greater than 70%, 80%, 90%, 95%, 98% or 99%, respectively with fragments of the Coversin protein sequence in Figure 2. Fragments with improved may, of course, be rationally designed by the systematic mutation or fragmentation of the wild type sequence followed by appropriate activity assays. Fragments may exhibit similar or greater affinity for C5, either the wild-type or polymorphic variant of C5 or both, and/or LTB4 as Coversin. These fragments may be of a size described above for fragments of the Coversin protein.

A functional equivalent used according to the invention may be a fusion protein, obtained, for example, by cloning a polynucleotide encoding the Coversin protein in frame to the coding sequences for a heterologous protein sequence. The term "heterologous", when used herein, is intended to designate any polypeptide other than the Coversin protein or its functional equivalent. Example of heterologous sequences, that can be comprised in the soluble fusion proteins either at N- or at C-terminus, are the following: extracellular domains of membrane-bound protein, immunoglobulin constant regions (Fc region), multimerization domains, domains of extracellular proteins, signal sequences, export sequences, or sequences allowing purification by affinity chromatography. Many of these heterologous sequences are commercially available in expression plasmids since these sequences are commonly included in the fusion proteins in order to provide additional properties without significantly impairing the specific biological activity of the protein fused to them [29]. Examples of such additional properties are a longer lasting half-life in body fluids, the extracellular localization, or an easier purification procedure as allowed by a tag such as a histidine, GST, FLAG, avidin or HA tag.

The Coversin protein and functional equivalents thereof, may be prepared in recombinant form by expression in a host cell. Such expression methods are well known to those of skill in the art and are described in detail by [30] and [31]. Recombinant forms of the Coversin protein and functional equivalents thereof are preferably unglycosylated.

The proteins and fragments of the present invention can also be prepared using conventional techniques of protein chemistry. For example, protein fragments may be prepared by chemical synthesis. Methods for the generation of fusion proteins are standard in the art and will be known to the skilled reader. For example, most general molecular biology, microbiology recombinant DNA technology and immunological techniques can be found in [30] or [32].

### Modes of administration

Coversin and its functional equivalents do not require a medical professional for administration to be carried out, and these molecules are rapidly absorbed. Many recombinant antibodies are absorbed very slowly and as a result need to be infused over long periods (e.g. intravenously). The administration of such molecules thus also requires a medical professional. Thus, as well as having the advantage of being more effective at inhibiting the activation of the complement pathways in subjects with a C5 polymorphism, Coversin also possesses the advantage of being easier to administer than other agents such as antibodies like eculizumab.

The agent is administered in a therapeutically or prophylactically effective amount. The term "therapeutically effective amount" refers to the amount of agent needed to treat the complement-mediated disease or disorder, as defined elsewhere herein. The term "prophylactically effective amount" used herein refers to the amount of agent needed to prevent complement-mediated disease or disorder as defined elsewhere herein. Preferably, the dose of the agent is sufficient to bind as much available C5 as possible in the subject, more preferably, all available C5. The dose of the agent may alternatively be sufficient to bind as much available LTB4 as possible in the subject, more preferably, all available LTB4. In some aspects, the dose of the agent is sufficient to binds as much available C5 and LTB4 as possible, for example all available C5 and LTB4. The dose of the agent supplied may be at least 1x or 1.5 times or twice the molar dose needed to bind all available C5 and/or LTB4 in the subject. The dose of the agent supplied may be e.g. 1, 1.5, 2, 2.5 times, 3 times or 4 times the molar dose needed to bind all available C5 and/or LTB4 in the subject. Preferably, the dose is from 0.0001 mg/kg (mass of drug compared to mass of patient) to 20 mg/kg, preferably 0.001 mg/kg to 10 mg/kg, preferably 0.01 mg/kg to 2 mg/kg, preferably O.lmg/kg to 1mg/kg; alternatively 0.2mg/kg to 0.8mg/kg; alternatively 0.3mg/kg to 0.7mg/kg; alternatively 0.4mg/kg to 0.6mg/kg; for example 0.14mg/kg or 0.57mg/kg. The therapeutically or prophylactically effective amount can additionally be defined in terms of the inhibition of terminal complement, for example, an amount that means that terminal complement activity (TCA) is reduced by at least 10, 20, 30, 40, 50, 60, 70, 80, 90, 91, 92, 93, 94, 95, 96, 97, 98, 99, 100%, compared to terminal complement activity in the absence of treatment. Dose and frequency may be adjusted in order to maintain terminal complement activity at the desired level, which may be, for example 10% or less, for example 9, 8, 7, 6, 5, 4, 3, 2, 1% or less compared to terminal complement activity in the absence of treatment.

Where a dose is given, this relates to a dose of the agent which is a protein or functional equivalent thereof. Appropriate doses for an agent which is a nucleic acid molecule may be used to give rise to these levels.

Terminal complement activity can be measured by standard assays known in the art, e.g. using the Quidel CH₅₀ haemolysis assay and the sheep red blood cell lytic assay.

The frequency with which the dose needs to be administered will depend on the half-life of the agent involved. The Coversin protein or a functional equivalent thereof, may be administered as a continuous infusion, in bolus doses or on a daily basis, twice daily basis, or every two, three, four days, five, six, seven, 10, 15 or 20 days or more. As noted elsewhere, a particular advantage of the Coversin protein and its functional equivalents is the relative ease and rapidity with which it can be administered, and the fact that medical professionals are not required for administration.

Single or multiple doses may be administered. For example at least 2, 3, 4, 5, 6, 7, or 8 doses may be administered. Single doses are one embodiment. The exact dosage and the frequency of doses may also be dependent on the patient's status at the time of administration. Factors that may be taken into consideration when determining dosage include the need for treatment or prophylaxis, the severity of the disease state in the patient, the general health of the patient, the age, weight, gender, diet, time and frequency of administration, drug combinations, reaction sensitivities and the patient's tolerance or response to therapy. The precise amount can be determined by routine experimentation, but may ultimately lie with the judgement of the clinician.

The dosage regimen may also take the form of an initial "loading dose" followed by one or more subsequence "maintenance doses". In general, the loading dose will be greater than the maintenance dose. The loading dose may be 2, 5, 10 or more times greater than the maintenance dose. The loading dose may be administered as a single dose, or as one or more doses in a particular time frame. Typically, the loading dose will be 1, 2, 3, 4 or 5 doses administered in a single 24 hour period. The maintenance dose may be a lower dose that is repeated at regular intervals. The maintenance dose may be repeated at intervals, such as every 3, 4, 6, 8, 12, 24, or 48 hours. The precise regimen can be determined by routine experimentation, but may ultimately lie with the judgement of the clinician. The maintenance dose may be at least 20, 30, 40, 50, 60, 70, 80, 90 or 100% of the initial loading dose, or up to 20, 30, 40, 50, 60, 70, 80, 90 or 100% of the initial loading dose.

In a further embodiment the same dose is used throughout the course of treatment (e.g. daily).

The loading dose may be 0.0001mg/kg (mass of drug compared to mass of patient) to 20mg/kg, and the maintenance dose may be between 0.0001 mg/kg to 20mg/kg; alternatively the loading dose is 0.001 mg/kg to 10 mg/kg and the maintenance dose is 0.001 mg/kg to 10 mg/kg, alternatively the loading dose is 0.01 mg/kg to 2 mg/kg and the maintenance dose is 0.01mg/kg to 2mg/kg; alternatively the loading dose is O.lmg/kg to 1mg/kg and the maintenance dose is O.lmg/kg to 1mg/kg; alternatively the loading dose is O.lmg/kg to 1mg/kg and the maintenance dose is 0.05mg/kg to 0.5mg/kg; alternatively the loading dose is 0.2mg/kg to 0.8mg/kg and the maintenance dose is O.lmg/kg to 0.4mg/kg; alternatively the loading dose is 0.3mg/kg to 0.7mg/kg and the maintenance dose is 0.1mg/kg to 0.3mg/kg; alternatively the loading dose is 0.4mg/kg to 0.6mg/kg and the maintenance dose is O.lmg/kg to 0.2mg/kg for example where the loading dose is 0.57mg/kg and the maintenance dose is 0.14mg/kg.

The loading dose may be 0.0001mg/kg (mass of drug compared to mass of patient) to 20mg/kg, and the maintenance dose may be between 0.0001 mg/kg to 20mg/kg; alternatively the maintenance dose may be 0.001 mg/kg to 10 mg/kg, alternatively the maintenance dose may be 0.01mg/kg to 2mg/kg; alternatively the maintenance dose may be O.lmg/kg to 1mg/kg; alternatively the maintenance dose may be O.lmg/kg to 0.8mg/kg; alternatively the maintenance dose may be O.lmg/kg to 0.6mg/kg; alternatively the maintenance dose may be O.lmg/kg to 0.4mg/kg; alternatively the maintenance dose may be O.lmg/kg to 0.2mg/kg.

The loading dose may be 0.0001mg/kg (mass of drug compared to mass of patient) to 20mg/kg, and the maintenance dose may be between 0.0001 mg/kg to 20mg/kg; alternatively the loading dose may be 0.001 mg/kg to 10 mg/kg, alternatively the loading dose may be 0.01 mg/kg to 2 mg/kg; alternatively the loading dose may be O.lmg/kg to 1mg/kg; alternatively the loading dose may be 0.1mg/kg to 1mg/kg; alternatively the loading dose may be 0.2mg/kg to 0.8mg/kg; alternatively the loading dose may be 0.3mg/kg to 0.6mg/kg; alternatively the loading dose may be 0.4mg/kg to 0.6mg/kg. The agent will generally be administered in conjunction with a pharmaceutically acceptable carrier. The term "pharmaceutically acceptable carrier", as used herein, includes genes, polypeptides, antibodies, liposomes, polysaccharides, polylactic acids, polyglycolic acids and inactive virus particles or indeed any other agent provided that the carrier does not itself induce toxicity effects or cause the production of antibodies that are harmful to the individual receiving the pharmaceutical composition. Pharmaceutically acceptable carriers may additionally contain liquids such as water, saline, glycerol, ethanol or auxiliary substances such as wetting or emulsifying agents, pH buffering substances and the like. The pharmaceutical carrier employed will thus vary depending on the route of administration. Carriers may enable the pharmaceutical compositions to be formulated into tablets, pills, dragees, capsules, liquids, gels, syrups, slurries, suspensions to aid intake by the patient. A thorough discussion of pharmaceutically acceptable carriers is available in [33].

The agent is delivered systemically. The agent may be delivered by a parenteral route (*e.g.* by injection, either subcutaneously, intraperitoneally, intravenously or intramuscularly or delivered to the interstitial space of a tissue). Other modes of administration include oral and pulmonary administration, suppositories.

Preferably the agent is delivered via subcutaneous injection. In some embodiments this is via once daily subcutaneous injection, for example at an initial loading dose of between 0.0001mg/kg (mass of drug compared to mass of patient) to 20mg/kg, followed by once daily maintenance doses of between 0.0001mg/kg to 20mg/kg, or other doses disclosed elsewhere herein. Alternatively the agent may be delivered via subcutaneous injection every other day.

In a preferred embodiment the agent is delivered via once daily subcutaneous injection at an initial loading dose of 0.4mg/kg-0.6mg/kg (for example 0.57mg/kg) followed by once daily maintenance doses of 0.1mg/kg-0.2mg/kg (for example 0.14mg/kg).

Preferably the course of treatment is continued for at least 6 weeks, e.g. at least 7, 8, 9, 10, 15, 20, 25, 30, 35, 40, 50 weeks or at least 2, 3, 4, 5, 6, 7, 8, 9, 10 months, or at least 1, 2, 3 years. The course of treatment is preferably continued until the subject is no longer considered to be suffering from acute GVHD, or until the subject no longer requires treatment. The course of treatment may thus be administration of the agent (e.g. daily) for at least 7, 8, 9, 10, 15, 20, 25, 30, 35, 40, 50 weeks or at least 2, 3, 4, 5, 6, 7, 8, 9, 10 months, or at least 1, 2, 3 years. Daily administration may be continued for at least 2, 3, 4, 5, 6, 7, 8, 9, 10 weeks. The frequency of administration may be modified after at least 2, 3, 4, 5, 6, 7, 8, 9, 10 weeks, e.g. to once every two days.

The maintenance dose (e.g. a single daily maintenance dose) may remain constant throughout the course of treatment) or the maintenance dose (e.g. a daily maintenance dose) may be modified (e.g. increased or decreased) during the course of treatment. The maintenance dose may be modified in order to maintain terminal complement activity at a desired level, e.g. 10% or less compared to serum from said patient in the absence of treatment or compared to normal control serum. The or each maintenance dose may be continued for at least 1, 2, 3, 4, 5, 6, 7, 8, 9, 10 weeks, e.g. daily for at least 1, 2, 3, 4, 5, 6, 7, 8, 9, 10 weeks. The maintenance dose may be decreased as the subject's symptoms improve. The amount of agent or the frequency with which the agent is administered may be decreased as the subject's symptoms improve.

There may thus be an initial loading dose, followed by an initial maintenance dose (e.g. a daily initial maintenance dose) which may be a maintenance dose as defined above, and one or more further maintenance doses (e.g. a daily further maintenance dose), e.g. at least 2, 3, 4, 5 further maintenance doses.

The disclosure (not according to the invention) thus further comprises a method of treating acute GVHD in a subject comprising systemically administering an initial loading dose of the agent as defined above, and then systemically administering maintenance doses (e.g. daily maintenance doses) of the agent as defined above, wherein there is an initial maintenance dose and one or more further maintenance doses.

The invention thus further comprises an agent for use in a method of treating acute GVHD in a subject as defined in the claims, the method comprising systemically administering an initial loading dose of the agent as defined above, and then systemically administering maintenance doses (e.g. daily maintenance doses) of the agent as defined above, wherein there is an initial maintenance dose and one or more further maintenance doses.

The one or more further maintenance doses may be determined by testing the terminal complement activity in the subject (e.g. in a biological sample from the subject), and determining the further maintenance dose for said subject on the basis of the level of terminal complement activity and/or testing the subject's symptoms and determining the further maintenance dose for said subject on the basis of the subject's symptoms. The method may optionally further comprise administering said further maintenance dose. Said further dose may be calculated to be at a level that maintains terminal complement activity at the desired level.

Where a biological sample is taken, this may be blood, e.g. a whole blood or a serum sample. The method optionally further comprises the step of taking the sample, and further optionally comprises the step of determining the TCA of the sample.

The one or more further maintenance doses may be determined by testing the terminal complement activity in the subject (e.g. in a biological sample from the subject), and determining the further maintenance dose for said subject on the basis of the level of terminal complement activity and/or testing the subject's symptoms and determining the further maintenance dose for said subject on the basis of the subject's symptoms. The method may optionally further comprise administering said further maintenance dose. Said further dose may be calculated to be at a level that maintains terminal complement activity at the desired level.

In certain aspects, the desired complement activity level is 10% or less compared to serum from said subject in the absence of treatment or compared to normal control serum.

In certain aspects, if the TCA is higher than the desired level the maintenance dose is increased, and optionally wherein if TCA is less than 5, 4, 3, 2, 1% the dose is maintained or decreased.

In certain aspects, if the symptoms deteriorate the maintenance dose is increased, and optionally wherein if the symptoms improve the dose is maintained or decreased.

In some embodiments the subject is tested within one month of initiating the treatment, within two weeks of initiating the treatment, within a week of initiating the treatment. In other embodiments the subject is tested once a day or at least once a day, once a week, or at least once a week, once every two weeks or at least once every two weeks, once a month or once every two months.

Preferably the loading dose is 0.57mg/kg protein or functional equivalent and the maintenance dose is at least 0.15mg/kg (e.g. at least 0.2mg/kg, 0.15-0.6, 0.2-0.5 or 0.25-0.45mg/kg) or is up to 0.13mg/kg (e.g. up to 0.1mg/kg, 0.05-0.13, 0.075-0.1mg/kg) and optionally (i) that maintenance dose is continued for at least 2, 3, 4, 5, 6 weeks and/or (ii) treatment is continued for at least 6 weeks and/or (iii) treatment is continued daily for at least 3, 4, 5, 6 weeks.

Preferably the loading dose is 0.4-0.6 mg/kg protein or functional equivalent and the maintenance dose is 0.1-0.2 mg/kg, e.g. 0.14mg/kg, and (i) that maintenance dose is continued for at least 2, 3, 4, 5, 6 weeks and/or (ii) treatment is continued for at least 6 weeks and/or (iii) treatment is continued daily for at least 3, 4, 5, 6 weeks.

The present invention relates to agents for systemic administration. This means that the agent is present in the circulatory system so that the entire body is affected. This is in contrast with topical administration where the effect is generally local. The systemic presence of the agent can be determined functionally, e.g. on the basis of TCA in the serum of the subject, or directly (e.g. by ELISA or mass spectrometry).

The agent may be administered alone or as part of a treatment regimen also involving the administration of other drugs currently used in the treatment or prophylaxis of patients with acute GVHD, such as Cyclosporine A, Methotrexate, Tacrolimus and glucocorticoids such as prednisone. The agent, alone or with one or more other drug currently used in the treatment or prophylaxis of acute GVHD may be administered at least once before the transplant which gives rise to acute GVHD (e.g. at least one day, or one week or 2 weeks before the transplant), with the transplant or after the transplant (e.g. the initial administration of the agent is up to one day or one week or 2 weeks, or one month or 2 months after the transplant, or up to one day or one week or 2 weeks, or one month or 2 months after diagnosis of acute GVHD)

The agent may be administered simultaneously, sequentially or separately with the other drug(s). For example, the agent may be administered before or after administration of the other drug(s).

Various aspects and embodiments of the present invention will now be described in more detail by way of example.

### BRIEF DESCRIPTION OF FIGURES:

**Figure 1****:** Schematic diagram of classical and alternative pathways of complement activation. Enzymatic components, dark grey. Anaphylatoxins enclosed in starbursts.
**Figure 2****:** Primary sequence of Coversin. Signal sequence underlined. Cysteine residues in bold type. Nucleotide and amino acid number indicated at right.
**Figure 3****:** Effect of spiking serum from Patient BJ and normal control (NC) with Coversin and eculizumab at various concentrations
**Figure 4****:** Serum terminal complement activity shown from June 13th - July 12th.
**Figure 5****:** LDH in the patient over the course of various treatments.

### EXAMPLES

### Example 1 -Case Report

The patient was a boy of 4 years old who first presented in 2013 with intermittent abdominal pain and rectal bleeding and was subsequently diagnosed as having chronic granulomatous disease. He underwent haematopoietic stem cell bone marrow replacement from a well-matched donor in October 2013 and received cyclosporine and tacrolimus post operatively. His course was complicated by lower gastrointestinal (GI) bleeding and an initial diagnosis of graft versus host disease (GVHD) was made. Subsequently severe thrombocytopenia developed although ADAMTS 13 activity remained normal and antibodies were not detected. Renal function remained normal.

The patient was treated initially with rituximab and infliximab with little improvement. Eculizumab was started in April 2014 but there was no improvement in platelet levels and he continued to need infusion of 2 adult units of fresh platelets daily. Lower GI bleeding continued intermittently and upper GI endoscopy on 14th May revealed ongoing gastritis and duodenitis with thinning and patchy necrosis of the mucosa. Lower GI endoscopy as far as the splenic flexure showed tar-like stools but it was not possible to identify a definite bleeding site. It was considered to be too hazardous to proceed further and the procedure was terminated. Histology of upper GI biopsies confirmed the clinical findings and were considered to be consistent with graft versus host disease.

### Clinical Course

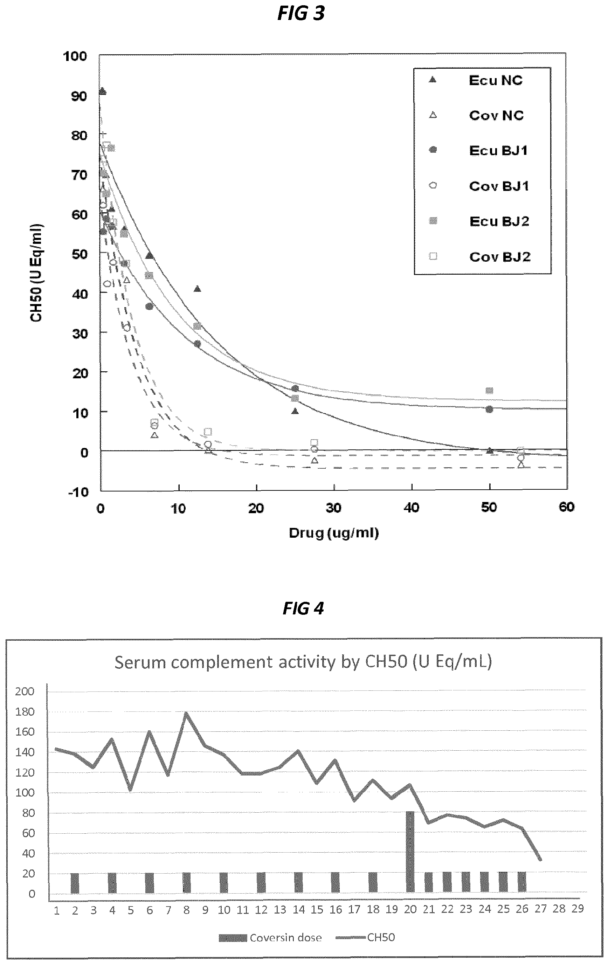

In late May 2014, after 2 months of weekly infusions of a full adult dose of eculizumab with no clinical improvement, genetic testing revealed that the patient had the sArg885His polymorphism. On 29th May 2014 a serum sample was tested in the Volution complement laboratory at the Haemostasis Research Unit (HRU) at University College, London (UCL) using the Quidel CH50 complement haemolysis assay and spiking with ascending doses of both Coversin and eculizumab. Pooled serum from normal subjects was used as control. The results of this are shown in Figure 3.

This demonstrated that Coversin at c. 15µg/mL, the known therapeutic concentration, completely ablated terminal complement activity (TCA) in both serum from BJ and in normal control serum. Eculizumab 50µg/mL ablated TCA in normal control serum but was unable to inhibit TCA beyond c. 80% at any concentration tested. During the eculizumab treatment there was a continuing clinical picture which included thrombocytopaenia, rectal bleeding and serum lactic dehydrogenase (LDH) fluctuating between c.900 and 2,000 IU/L.

A request was made by the responsible physician for Coversin to treat the patient and, following discussions with the patient's parents a limited supply of Coversin was provided for subcutaneous injection and eculizumab was discontinued.

The quantity of clinical grade Coversin available at the time was extremely limited and consisted of the residual from the PIa clinical trial for Coversin. After discussion, despite the limited availability of Coversin, it was decided to commence treatment on empirical grounds with an ablating dose of 0.57mg/kg and thereafter a daily maintenance dose of 25% of that. Treatment commenced on 1st June 2014 and the initial clinical response appeared to be encouraging with stabilisation of vital signs and reduced rectal bleeding. After 7 daily maintenance doses of Coversin, it was decided to space out the daily maintenance doses to alternate days in order to conserve the supply of Coversin as long as possible, although, at the start of treatment whilst the assay was in development, accurate CH50 assays were not available. By 4th July, 34 days after the start of Coversin treatment it was clear that, although there was clinical improvement compared with the period prior to Coversin, control of TCA was not complete and this was subsequently confirmed by retrospective CH50 assays. A decision was made to increase the dose of Coversin. A further ablating dose of 0.57mg/kg was given on 5th July and daily maintenance doses of 25% of that were resumed thereafter until Coversin ran out 6 days later.

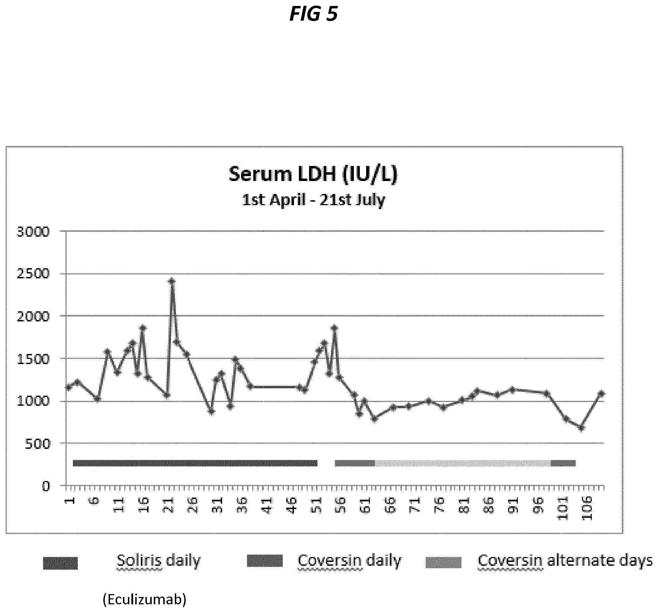

Figure 4 shows the effect of Coversin on CH50 activity from the time that reliable data became available and Figure 5 shows the effect of both Coversin and eculizumab on LDH from 1st April to 11th July. Following the last dose of Coversin on July 11th the patient's clinical status deteriorated rapidly and he died on 4th August, 24 days later.

The LDH results (Figure 5) between 1st April and 21st July indicate that for much of the period that the patient received eculizumab, terminal complement activity was uncontrolled and that lysis of both red cells and platelets was taking place. Following the introduction of Coversin on 1st June LDH fell from c.1800 to 789 IU/L. After reduction of the repeat dose from daily to alternate days LDH remained around 1,000 IU/L but with less variability than had been the case during the previous two months of eculizumab treatment. Once daily Coversin treatment was re-instated on 5th July LDH again fell to 688IU/L but rose again following the discontinuation of Coversin 6 days later.

The CH50 data indicate that daily dosing with Coversin at 25% of the ablating dose controlled TCA.

During the 58 days that the patient received Coversin there were no reported adverse events attributable to the drug and no injection site reactions. Low titre anti-Coversin antibodies (IgG) were detected after 14 days treatment but were non-neutralising.

### Discussion

This case shows that people of non-Japanese/Chinese origin may also be affected by the polymorphisms previously described only in these ethnic groups by Nishimura, J et al., New Engl J. Med., 30;7: 632-639 (2014).

Elevated serum LDH is an early indicator of TMAs and occult malignancy and is a good indicator of response to therapy [34]. In this case, although it never fully returned to normal levels, there was a clear indication that during the two periods when an adequate dose of Coversin was being given it fell suggesting that red cell and platelet destruction and mucosal damage were diminishing. By contrast, during the two months of eculizumab treatment, the LDH remained generally above 1,000 IH/L and extremely variable.

Coversin completed a single dose Phase I clinical trial in 2014 in normal volunteers by subcutaneous injection. At a dose of 0.57mg/kg the drug caused total blockade of complement haemolytic activity in all subjects at 12 hours post-dose returning to ∼50% activity by 48 hours.

### Methods-in vitro inhibition of C5 activity

The Quidel Microvue CH50 Eq enzyme immunoassay (cat #A018) is for *in vitro* measurement of total classical pathway activity in human serum. http://www.quidel.com/sites/quidel.com/files/product/documents/a018_microvue_ch50_eq _english_1.pdf

The kit provides a direct measure of the terminal complement complex (TCC) formation under standard conditions. Measurement of CH50 with the kit has 3 steps:
1. Activation of the classical complement pathway in undiluted serum resulting in formation of TCC.
2. Dilution of serum and addition to microassay wells coated with an antibody that captures TCC.
3. Quantification of captured TCC with and anti-TCC horse radish peroxidase (HRP) conjugated antibody.

Colour intensity on addition of substrate is proportional to the concentration of TCC present in each reaction. Using the kit standard curve (determined during each assay) assay results are expressed in CH50 unit equivalents per millilitre (CH50 U Eq/ml).

The linear range for the kit is 30-310 CH50 U Eq/ml.

According to the manufacturers the cut off for normality determined from 234 individual human samples is 70 CH50 U Eq/ml.

### Reagents & Samples

Sample Preparation: Serum was prepared by collecting blood into plain glass or SST Vacutainer tubes (or equivalent) and allowing it to clot for 1 hour, before centrifugation at 1500g for 10 minutes. The serum was separated immediately (avoiding contamination with any blood cells) and stored in screw cap cryotubes (approximately 0.5ml aliquots) at -70°C.

Coversin: Frozen 10.9mg/ml solution at -70°C. Dilute lOuL in 90uL normal control or patient serum to give a final concentration of 1.09mg/ml. Dilute lOuL in 90uL autologous serum to give a final concentration of 109ug/ml. Double dilute in autologous serum to achieve a final concentration range of: 0.4 - 54.5ug/ml.

Eculizumab: Frozen solution of 10mg/ml. Dilute lOuL in 90uL normal control or patient serum to give a final concentration of 1mg/ml. Dilute lOuL in 90uL autologous serum to give a final concentration of 100ug/ml. Double dilute in autologous serum to achieve a final concentration range of: 0.4 - 50ug/ml.

Buffer: Phosphate Buffered Saline (0.01M phosphate buffer, 0.0027M potassium chloride, 0.137M sodium chloride, pH7.4).

Coversin, Eculizumab, or buffer (control), are spiked into serum according to the procedure above to achieve a range of final concentrations. These are then assayed for CH50 Equivalent activity using the Quidel CH50 kit, using duplicate wells.

CH50 values are calculated from the calibration curve provided with the kit and plotted as raw CH50 values against C5 inhibitor concentration. The CH50 results are calculated at each C5 inhibitor concentration as a percentage of the CH50 concentration of the relevant buffer control and plotted against inhibitor concentration.

Repeat the experiment on separate days to obtain 3 measurements in each patient and in a single normal control. This provides an estimate of between experiment variability.

Repeat the experiment on separate days in single experiments on 6 different normal controls. This provides an estimate of between subject responsiveness (and avoids the risk of using a single subject who may have an unknown C5 mutation or polymorphism).

The highest dose of each drug to whole serum was added and then two-fold serial dilutions were made in whole serum. One replicate was used for each drug dose.

After serial dilution the serum was activated and assayed in accordance with the instructions for the Quidel CH50 kit.

CH50 U Eq/ml were calculated in comparison with the kit standards and plotted against drug concentration for each of the three serum samples and two drug treatments. They were also plotted as a percentage of the CH50 value of the relevant buffer only control.

### REFERENCES:

[1] Billingham RE. The biology of graft-versus-host reactions. Harvey Lect. 1966-1967. 62:21-78
[2] http://emedicine.medscape.com/article/429037-clinical#b4
[3] Weston-Davies W, et. al Blood (ASH Annual Meeting Abstracts) 2014; 124:4280
[4] Pfarr N, et al. J Immunol. 174(7):4172-7. (2005) Erratum in: J Immunol.;182(8):5152
[5] Delgado-Cerviño E, Fontán G, Lopez-Trascasa M. Mol Immunol. 42(1):105-11. (2005)
[6] Halangk J, et al., J Hepatol. 49(3):339-45 (2008)
[7] Nishimura, J et al., New Engl J. Med., 30;7: 632-639 (2014)
[8] Wang X, Fleischer DT, Whitehead WT, Haviland DL, Rosenfeld SI, Leddy JP, Snyderman R, Wetsel RA. J Immunol. 154(10):5464-71. (1995)
[9] Jakowski et al., Clin Diagn Lab Immunol. 6(1): 137-139 (1999)
[10] Freslund et al., Nature Immunology 9, 753 - 760 (2008)
[11] Hobart, M. J., et al., Annals of Human Genetics 45.1 (1981): 1-4.
[12] Jakowski et al., Clin Diagn Lab Immunol. 6(1): 137-139 (1999)
[13] Sahu et al., Immunopharmacology, 49:133-148 (2000).
[14] Ricklin D & Lambris J, Nature Biotechnology, 25: 1265-1275 (2007)
[15] WO 2007/028968
[16] WO 2008/029169
[17] WO 2008/029167
[18] WO 2011/083317
[19] WO 2004/106369
[20] WO 2009/098454
[21] Guo, R.F. and P.A. Ward, Annu Rev Immunol, 2005, 23: p. 821-52
[22] Neumann, E., et al., Arthritis Rheum, 2002. 46(4): p. 934-45
[23] Williams, A.S., et al., Arthritis Rheum, 2004, 50(9): p. 3035-44
[24] Quigg, R.J., Curr Dir Autoimmun, 2004. 7: p. 165-80
[25] Papagianni, A.A., et al., Nephrol Dial Transplant, 2002, 17(1): p. 57-63
[26] He, C., et al., J Immunol, 2005. 174(9): p. 5750-7
[27] Mead, R.J., et al., J Immunol, 2002. 168(1): p. 458-65
[28] Nakashima, S., et al., J Immunol, 2002. 169(8): p. 4620-7
[29] Terpe K, Appl Microbiol Biotechnol, 60: 523-33, 2003
[30] Sambrook *et al* (2000)
[31] Fernandez & Hoeffler (1998)
[32] Ausubel *et al.* (1991)
[33] Remington's Pharmaceutical Sciences; Mack Pub. Co., N.J. 1991
[34] Pirrotta MT, Bucalossi A. Thrombotic microangiopathy and occult neoplasia. Cardiovasc Hematol Disord Drug Targets. 2010 Jun;10(2):87-93.

## Claims

1. An agent for use in treating or preventing acute GVHD in a subject, wherein the subject has not been treated with eculizumab, wherein the agent is administered systemically and wherein the agent is:
(i) a protein comprising amino acids 19 to 168 of the amino acid sequence in SEQ ID NO: 2 or a functional equivalent of this protein; or
(ii) a nucleic acid molecule encoding a protein as recited in (i),
wherein the functional equivalent is:
(a) a homologue of a protein as defined in (i) comprising or consisting of a sequence with greater than 80% identity to the sequence of amino acids 19 to 168 or amino acids 1 to 168 of the amino acid sequence SEQ ID NO: 2; or
(b) a fragment of a protein, wherein the protein has the amino acid sequence of SEQ ID NO: 2, or a fragment of a homologue, wherein the homologue has greater than 80% identity to the sequence of amino acids 19 to 168 or amino acids 1 to 168 of the amino acid sequence SEQ ID NO: 2,
wherein said functional equivalent retains the ability to bind complement C5 and to prevent the cleavage of complement C5 by C5 convertase into complement C5a and complement C5b-9.

2. The agent for use according to claim 1, wherein the subject does not have or has not been tested for a complement C5 polymorphism, wherein the C5 polymorphism is Arg885Cys or Arg885His.

3. The agent for use according to claim 1 or claim 2, wherein the subject does not have or has not been tested for resistance to eculizumab.

4. The agent for use according to any one of claims 1 to 3, wherein the subject has not been selected for treatment with the agent on the basis of decreased effectiveness in the subject of a different agent, such as anti C5 antibody.

5. The agent for use according to of any one of claims 1 to 4, wherein the subject has a GVHD symptom at stage +, ++, +++ or ++++ and/or the subject has a clinical grading of I, II, III or IV,

6. The agent for use according to any one of claims 1 to 5, wherein the subject has tissue damage, e.g. internal (such as intestinal) tissue damage arising from the GVHD.

7. The agent for use according to any one of claims 1 to 6, wherein the subject has acute GVHD following allogenic haematopoietic cell transplant (HCT), and/or has received no GVHD prophylaxis.

8. The agent for use according to any one of claims 1 to 7, wherein the subject is at least 5 years old, optionally wherein the subject is an adult.

9. The agent for use according to any one of claims 1 to 8, wherein the subject has received HLA-nonidentical stem cells.

10. The agent for use according to any one of claims 1 to 9, wherein the subject has acute GVHD following solid-organ transplantation, or following transfusion of unirradiated blood products.

11. The agent for use according to any one of claims 1 to 10, wherein the subject has acute GVHD after autologous or syngeneic HCT.

12. The agent for use according to any one of claims 1 to 11, wherein the subject has hyperacute GVHD.

13. The agent for use according to any one of claims 1 to 12, wherein the treatment is continued
i. for at least 6 weeks, and/or
ii. until the subject is no longer considered to be suffering from acute GVHD, and/or
iii. until the subject no longer requires treatment.

## Patentansprüche

1. Mittel zur Verwendung bei der Behandlung oder Vorbeugung von akuter GvHD bei einem Subjekt, wobei das Subjekt nicht mit Eculizumab behandelt worden ist, wobei das Mittel systemisch verabreicht wird und wobei das Mittel Folgendes ist:
(i) ein Protein, das die Aminosäuren 19 bis 168 der Aminosäuresequenz in SEQ ID NO: 2 umfasst oder ein funktionelles Äquivalent dieses Proteins; oder
(ii) ein Nukleinsäuremolekül, das für ein Protein kodiert, wie in (i) aufgeführt,
wobei das funktionale Äquivalent Folgendes ist:
(a) ein Homolog eines Proteins wie in (i) definiert, das eine Sequenz mit mehr als 80 % Identität mit der Sequenz der Aminosäuren 19 bis 168 oder der Aminosäuren 1 bis 168 der Aminosäuresequenz SEQ ID NO: 2 umfasst oder daraus besteht; oder
(b) ein Fragment eines Proteins, wobei das Protein die Aminosäuresequenz von SEQ ID NO: 2 aufweist, oder ein Fragment eines Homologs, wobei das Homolog mehr als 80 % Identität mit der Sequenz der Aminosäuren 19 bis 168 oder der Aminosäuren 1 bis 168 der Aminosäuresequenz SEQ ID NO: 2 aufweist,
wobei das funktionelle Äquivalent die Fähigkeit behält, Komplement C5 zu binden und die Spaltung von Komplement C5 durch die C5-Konvertase in Komplement C5a und Komplement C5b-9 zu verhindern.

2. Mittel zur Verwendung nach Anspruch 1, wobei das Subjekt keinen komplementären C5-Polymorphismus hat oder nicht darauf getestet wurde, wobei der C5-Polymorphismus Arg885Cys oder Arg885His ist.

3. Mittel zur Verwendung nach Anspruch 1 oder 2, wobei das Subjekt keine Resistenz gegen Eculizumab aufweist oder nicht auf Resistenz getestet wurde.

4. Mittel zur Verwendung nach einem der Ansprüche 1 bis 3, wobei das Subjekt nicht für die Behandlung mit dem Mittel auf der Grundlage einer verringerten Wirksamkeit eines anderen Mittels, wie beispielsweise eines Anti-C5-Antikörpers, bei dem Subjekt ausgewählt worden ist.

5. Mittel zur Verwendung nach einem der Ansprüche 1 bis 4, wobei das Subjekt ein GvHD-Symptom im Stadium +, ++, +++ oder ++++ aufweist und/oder das Subjekt eine klinische Einstufung von I, II, III oder IV hat.

6. Mittel zur Verwendung nach einem der Ansprüche 1 bis 5, wobei das Subjekt eine Gewebeschädigung aufweist, z. B. eine interne (wie etwa intestinale) Gewebeschädigung, die von der GvHD herrührt.

7. Mittel zur Verwendung nach einem der Ansprüche 1 bis 6, wobei das Subjekt akute GvHD nach einer allogenen hämatopoetischen Zelltransplantation (HCT) hat und/oder keine GvHD-Prophylaxe erhalten hat.

8. Mittel zur Verwendung nach einem der Ansprüche 1 bis 7, wobei das Subjekt mindestens 5 Jahre alt ist, wobei das Subjekt gegebenenfalls ein Erwachsener ist.

9. Mittel zur Verwendung nach einem der Ansprüche 1 bis 8, wobei das Subjekt HLA-nichtidentische Stammzellen erhalten hat.

10. Mittel zur Verwendung nach einem der Ansprüche 1 bis 9, wobei das Subjekt nach einer Transplantation fester Organe oder nach einer Transfusion von unbestrahlten Blutprodukten eine akute GvHD hat.

11. Mittel zur Verwendung nach einem der Ansprüche 1 bis 10, wobei das Subjekt akute GvHD nach autologer oder syngener HCT hat.

12. Mittel zur Verwendung nach einem der Ansprüche 1 bis 11, wobei das Subjekt eine hyperakute GvHD hat.

13. Mittel zur Verwendung nach einem der Ansprüche 1 bis 12, wobei die Behandlung fortgesetzt wird
i. über mindestens 6 Wochen und/oder
ii. bis das Subjekt nicht mehr an akuter GvHD leidet, und/oder
ii. bis das Subjekt keine Behandlung mehr benötigt.

## Revendications

1. Agent pour une utilisation dans le traitement ou la prévention d'une GVHD aiguë chez un sujet, le sujet n'ayant pas été traité avec l'éculizumab, l'agent étant administré par voie systémique, et l'agent étant :
(i) une protéine comprenant les acides aminés 19 à 168 de la séquence d'acides aminés de SEQ ID NO: 2 ou un équivalent fonctionnel de cette protéine ; ou
(ii) une molécule d'acide nucléique codant pour une protéine telle que mentionnée en (i),
l'équivalent fonctionnel étant :
(a) un homologue d'une protéine telle que définie en (i) comprenant, ou en étant constituée, une séquence ayant une identité supérieure à 80 % avec la séquence d'acides aminés 19 à 168 ou les acides aminés 1 à 168 de la séquence d'acides aminés SEQ ID NO: 2 ; ou
(b) un fragment d'une protéine, la protéine ayant la séquence d'acides aminés de SEQ ID NO: 2, ou un fragment d'un homologue, l'homologue ayant une identité supérieure à 80 % avec la séquence d'acides aminés 19 à 168 ou les acides aminés 1 à 168 de la séquence d'acides aminés SEQ ID NO: 2,
ledit équivalent fonctionnel conservant la capacité de se lier au C5 du complément et de prévenir le clivage du C5 du complément par la C5 convertase en le C5a du complément et le C5b-9 du complément.

2. Agent pour une utilisation selon la revendication 1, le sujet n'ayant pas ou n'ayant pas été testé pour un polymorphisme C5 du complément, le polymorphisme C5 étant Arg885Cys ou Arg885His.

3. Agent pour une utilisation selon la revendication 1 ou la revendication 2, le sujet n'ayant pas ou n'ayant pas été testé pour une résistance à l'éculizumab.

4. Agent pour une utilisation selon l'une quelconque des revendications 1 à 3, le sujet n'ayant pas été choisi pour traitement avec l'agent sur la base d'une efficacité diminuée chez le sujet d'un agent différent tel qu'un anticorps anti-C5.

5. Agent pour une utilisation selon l'une quelconque des revendications 1 à 4, le sujet ayant un symptôme GVHD au stade +, ++, +++ ou ++++ et/ou le sujet ayant un grade clinique I, II, III ou IV.

6. Agent pour une utilisation selon l'une quelconque des revendications 1 à 5, le sujet présentant une lésion tissulaire, par exemple une lésion d'un tissu interne (tel qu'un tissu intestinal) due à la GVHD.

7. Agent pour une utilisation selon l'une quelconque des revendications 1 à 6, le sujet ayant une GVHD aiguë après une transplantation de cellules hématopoïétiques allogènes (HCT) et/ou n'a pas reçu une prophylaxie contre la GVHD.

8. Agent pour une utilisation selon l'une quelconque des revendications 1 à 7, le sujet étant âgé d'au moins 5 ans, éventuellement le sujet étant un adulte.

9. Agent pour une utilisation selon l'une quelconque des revendications 1 à 8, le sujet ayant reçu des cellules souches HLA-non identiques.

10. Agent pour une utilisation selon l'une quelconque des revendications 1 à 9, le sujet ayant une GVHD aiguë après transplantation d'un organe solide, ou après transfusion de produits sanguins non irradiés.

11. Agent pour une utilisation selon l'une quelconque des revendications 1 à 10, le sujet ayant une GVHD aiguë après une HCT autologue ou syngénique.

12. Agent pour une utilisation selon l'une quelconque des revendications 1 à 11, le sujet ayant une GVHD hyperaiguë.

13. Agent pour une utilisation selon l'une quelconque des revendications 1 à 12, le traitement se poursuivant
i. pendant au moins 6 semaines, et/ou
ii. jusqu'à ce que le sujet ne soit plus considéré comme souffrant d'une GVHD aiguë, et/ou
iii. jusqu'à ce que le sujet n'exige plus de traitement.
